# EUROPÄISCHE PATENTSCHRIFT

(11) **EP 0 544 152 B1**
(45) Veröffentlichungstag und Bekanntmachung des Hinweises auf die Patenterteilung: **11.09.1996**
(21) Anmeldenummer: 92119334.8
(22) Anmeldetag: 12.11.1992
(51) Int. Cl.: C07D 215/56, A01N 43/42, C07D 409/12

(54) **Herbizide Chinolin-3-carbonsäureamide**
Herbicidal quinoline-3-carbonamides
Quinoléine-3-carbonamides herbicides

(30) Priorität: 26.11.1991 DE 4138820
(43) Veröffentlichungstag der Anmeldung: 02.06.1993
(73) Patentinhaber: BASF Aktiengesellschaft, 67063 Ludwigshafen (DE)
(72) Erfinder: von Deyn, Wolfgang, Dr., W-6730 Neustadt (DE); Theobald, Hans, Dr., W-6703 Limburgerhof (DE); Nuebling, Christoph, Dr., W-6733 Hassloch (DE); Kardorff, Uwe, Dr., W-6800 Mannheim 1 (DE); Walter, Helmut, Dr., W-6719 Obrigheim (DE); Westphalen, Karl-Otto, Dr., W-6720 Speyer (DE); Kappe, Thomas, Prof. Dr., A-8010 Graz (AT); Gerber, Matthias, Dr., W-67117 Limburgerhof (DE)

(56) Entgegenhaltungen:
- EP-A- 0 459 561

## Beschreibung

Die vorliegende Erfindung betrifft Chinolin-3-carbonsäureamide der allgemeinen Formel I 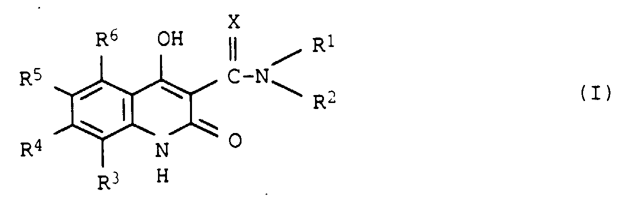 in der die Substituenten folgende Bedeutung haben:
- R¹: Wasserstoff, C₁-C₂₅-Alkyl, C₃-C₂₅-Alkenyl, C₃-C₂₅-Alkinyl, C₃-C₈-Cycloalkyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
- R²: Wasserstoff, Hydroxyl, C₁-C₈-Alkoxy, C₃-C₈-Alkenyloxy Di-C₁-C₄-Alkylamino, C₁-C₂₅-Alkyl, C₃-C₂₅-Alkenyl, C₃-C₂₅-Alkinyl, C₃-C₈-Cycloalkyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können:
C₁-C₄-Alkyl, C₁-C₄-Alkoxy, C₁-C₄-Halogenalkyl, C₁-C₄-Halogenalkoxy, Halogen, Cyano oder Nitro;
- R²: ferner substituiertes C₁-C₈-Alkoxy, C₃-C₈-Alkenyloxy, Di-C₁-C₄-Alkylamino, C₁-C₂₅-Alkyl, C₃-C₂₅-Alkenyl, C₃-C₂₅-Alkinyl oder C₃-C₈-Cycloalkyl, wobei diese Gruppen durch ein bis fünf Halogenatome und/oder ein bis zwei 5- bis 6-gliedrige heterocyclische, aliphatische oder aromatische Reste, enthaltend ein bis drei Heteroatome, ausgewählt aus der Gruppe Sauerstoff, Schwefel oder Stickstoff, substituiert sind, die ein oder zwei der folgenden Substituenten tragen können: Halogen, C₁-C₃-Alkyl oder C₁-C₃-Alkoxy;
oder
- R¹, R²: gemeinsam eine Alkylenkette mit 4 bis 7 Gliedern, welche durch Sauerstoff, Schwefel oder N-Methyl unterbrochen sein kann;
- R³-R⁶: Wasserstoff, C₁-C₄-Alkyl, C₁-C₄-Alkoxy, C₁-C₄-Halogenalkyl, C₁-C₄-Halogenalkoxy, C₁-C₄-Alkylthio, C₁-C₄-Halogenalkylthio, Halogen, Cyano oder Nitro;
- X: Sauerstoff oder Schwefel
oder deren umweltverträgliche Salze;
mit der Maßgabe, daß R² nicht Wasserstoff, C₁-C₃-Alkyl, n-Butyl, 3-Methylbutyl, Cyclohexyl, Hexyl, Heptyl, Octyl, 2-Chlorbenzyl, 3-(Dimethylamino)propyl, 3-(Diethylamino)propyl, 2-Morpholinoethyl und 2-(3,4-Dimethoxyphenyl)ethyl bedeutet, wenn R¹ und R³ bis R⁶ Wasserstoff und X Sauerstoff bedeuten und R² nicht Benzyl bedeutet, wenn R¹ Methyl, R³ bis R⁶ Wasserstoff und X Sauerstoff bedeutet, sowie R¹ und R² nicht gemeinsam für Morpholino stehen, wenn R³ bis R⁶ Wasserstoff und X Sauerstoff bedeuten. Ausgenommen sind ferner die Verbindungen 7-Chlor-2,4-dioxo-3-(1,2,3,4-tetrahydrochinolin)hydroxamsäure und 7-Chlor-5-iod-2,4-dioxo-3-(1,2,3,4-tetrahydrochinolin)hydroxamsäure.

Außerdem betrifft die Erfindung herbizide Mittel, welche die Verbindungen I als wirksame Substanzen enthalten, sowie die herbizide Verwendung von Chinolin-3-carbonsäureamiden der Formel I, einschließlich der per Disclaimer ausgenommenen Verbindungen.

Die Verbindungen der allgemeinen Formel I können in folgenden tautomeren Formen vorliegen, die ebenfalls von der Erfindung umfaßt werden.

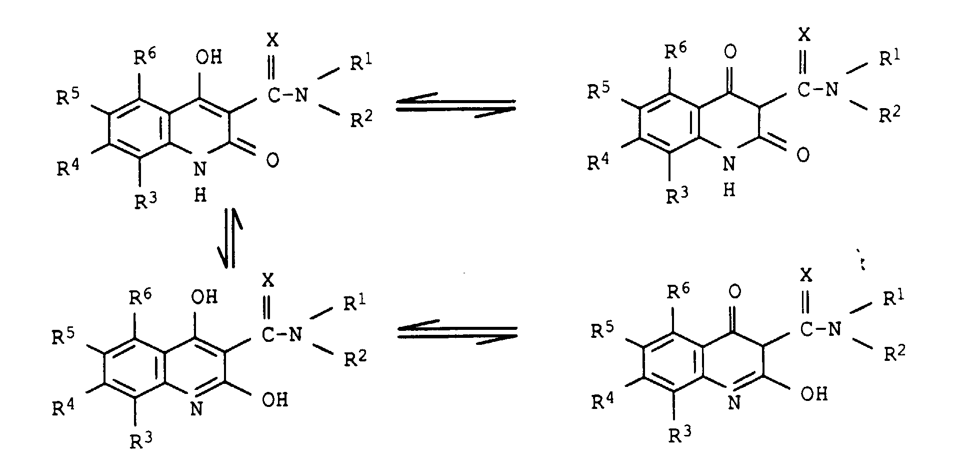

Aus Chemical Abstracts, Band 113, Nr. 211864z sind 4-Hydroxycarbostyrile mit entzündungshemmenden und antiallergischen Eigenschaften bekannt.

Außerdem sind Chinolin-3-carbonsäureamide mit gerinnungshemmenden (Khim.-Farm. Zh., 24(4), 31 (Russ), 1990)und antibakteriellen (Chemical Abstracts 70, Nr. 67681x) Eigenschaften, sowie Derivate, die als Lokalanästhetica wirken (Farm. Zh. (Kiev) (2), 78,1991), bekannt.

Aufgabe der vorliegenden Erfindung war es, neue herbizid wirksame Chinolin-3-carbonsäureamide und Verfahren zu deren Herstellung, bereitzustellen.

Demgemäß wurden die eingangs definierten Chinolin-3-carbonsäureamide I, Verfahren zu ihrer Herstellung und herbizide Mittel, die die Verbindungen I enthalten, gefunden. Gegenstand der Erfindung sind auch Salze der Verbindungen I.

Man erhält die Chinolin-3-carbonäureamide I beispielsweise dadurch, daß man einen Chinolin-3-carbonsäureester der allgemeinen Formel IIa in an sich bekannter Weise (Khim.-Farm. zh., Band 24, Heft 4, S. 31 und 32 (russ) ≙ S. 257-259 (engl.), 1990), gegebenenfalls in einem organischen Lösungsmittel, mit einem substituierten Amin der Formel III umsetzt:

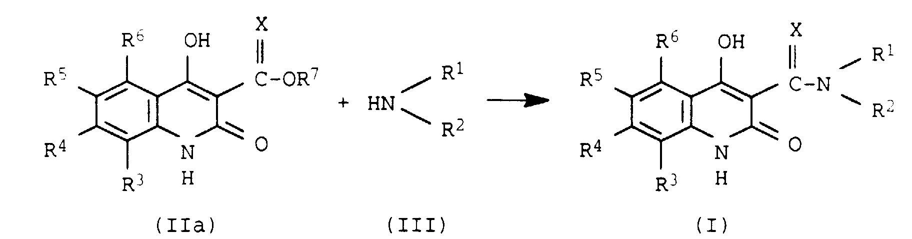

R⁷ in der Formel IIa steht für eine niedermolekulare Alkylgruppe, vorzugsweise C₁-C₄-Alkyl wie Methyl, Ethyl, Propyl, 1-Methylethyl, Butyl, 1-Methyl-propyl, 2-Methylpropyl oder 1,1-Dimethylethyl.

Die Umsetzung wird üblicherweise bei Temperaturen von 20°C bis 250°C, vorzugsweise 100°C bis 180°C in einem inerten organischen Lösungsmittel durchgeführt.

Als Lösungs- oder Verdünnungsmittel eignen sich beispielsweise aliphatische, alicyclische und aromatische Kohlenwasserstoffe wie Pentan, Hexan, Cyclohexan, Benzol, Toluol, Xylole; Ether wie Diethyl-, Di-n-butylether, Methyltert.-butylether, Dimethoxyethan, Ethylenglykoldimethylether, Tetrahydrofuran und Dioxan; Alkohole wie Methanol, Ethanol, Propanol und Butanol; aprotische dipolare Lösungsmittel wie Dimethylformamid, Dimethylsulfoxid oder Pyridin.

Es können auch Gemische dieser Stoffe als Lösungs- und Verdünnungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe, vorzugsweise das Amin, in einem Überschuß von 0,1 bis 10 mol-Äq. zu verwenden.

Die Umsetzung wird im allgemeinen bei Atmosphärendruck ausgeführt. Es kann jedoch, je nach Art des verwendeten Amins oder Lösungsmittels, vorteilhaft sein, die Umsetzung unter erhöhtem Druck, insbesondere unter autogen erhöhtem Druck in einem Autoklaven durchzuführen.

Weiterhin erhält man die Verbindungen I aus den Chinolin-3-carbonsäuren IIb indem man IIb zunächst in an sich bekannter Weise in das Halogenid oder eine andere aktivierte Form der Carbonsäurefunktion überführt und diese Derivate anschließend mit einem Amin III amidiert.

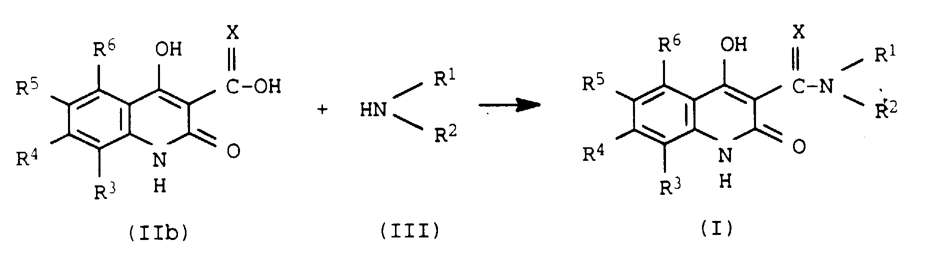

Aktivierte Formen der Carbonsäuren sind neben Halogeniden wie insbesondere den Chloriden und den Bromiden, beispielsweise auch die Imidazolide. Im allgemeinen werden die Halogenide bevorzugt.

Man erhält sie durch Umsetzung der Carbonsäuren IIb mit einem Halogenierungsmittel wie Phosgen, Thionylchlorid, Phosphoroxychlorid, Phosphortri- oder -pentachlorid.

Die anschließende Amidierung wird bei Temperaturen von (-20) bis 80°C, vorzugsweise 0 bis 30°C in einem inerten organischen Lösungsmittel durchgeführt.

Für diese Umsetzung eignen sich insbesondere Kohlenwasserstoffe wie Benzol oder Toluol, Halogenkohlenwasserstoffe wie Dichlormethan und Ether wie Diethylether und tert.-Butylmethylether als Lösungsmittel.

Da bei der Amidierung mit Säurehalogeniden Halogenwasserstoff gebildet wird, setzt man zur Steigerung der Ausbeute zweckmäßigerweise das Amin III im Überschuß ein oder setzt ein säurebindendes Mittel, beispielsweise Triethylamin, zu.

Vorteilhaft kann die Darstellung der Chinolin-3-carbonsäureamide I aus den Carbonsäuren IIb auch in einer Stufe durchgeführt werden. Dazu wird die Carbonsäure IIb mit einem Amin III in Gegenwart eines wasserentziehenden Mittels, z.B. Propanphosphonsäureanhydrid oder Dicyclohexylcarbodiimid, bei Temperaturen von 0 bis 50°C, bevorzugt 5 bis 25°C, in einem inerten Lösungsmittel wie Dichlormethan, Tetrahydrofuran, Toluol oder Ethylacetat in an sich bekannter Weise (WO 90 15052) zur Reaktion gebracht.

Ferner erhält man die Verbindungen der Formel I, in der X Sauerstoff und R¹ Wasserstoff bedeuten, in an sich bekannter Weise (J. Org. Chem. Band 44, S. 4877 ff (1979)) durch Umsetzung eines 2,4-Dihydroxychinolins IV mit Isocyanaten der Formel V.

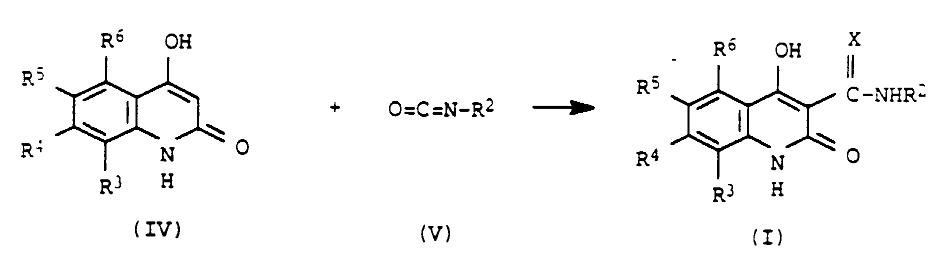

Die Chinolin-3-carbonsäuren und -ester der allgemeinen Formel II sind bekannt oder nach bekannten Methoden herstellbar (Australian J. Chem. Band 7, S. 348, S. 368 (1954)).

Die 2,4-Dihydroxychinoline IV sind ebenfalls bekannt oder können nach bekannten Methoden, z.B. wie in Monatsh. Chem., Band 96, 418 (1965)) hergestellt werden.

Die für die Umsetzungen benötigten Amine der Formel III und Isocyanate der Formel V sind bekannt, kommerziell erhältlich oder lassen sich nach allgemein bekannten chemischen Verfahren herstellen. Im Hinblick auf die bestimmungsgemäße Verwendung der Chinolin-3-carbonsäureamide der allgemeinen Formel I kommen als Substituenten folgende Reste in Betracht:
R¹
   Wasserstoff,
   unverzweigtes oder verzweigtes C₁-C₂₅-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl, 1-Ethyl-2-methyl-propyl, Heptyl, 1-Methylheptyl, Octyl, Nonyl, Decyl, Undecyl, Dodecyl, Tridecyl, Tetradecyl, Pentadecyl, Hexadecyl, Heptadecyl, Octadecyl, Nonadecyl, Arachidyl, Heneicosanyl, Docosyl, Tricosyl, Tetracosyl und Pentacosyl;
   insbesondere C₁-C₁₅-Alkyl, beispielsweise C₁-C₆-Alkyl wie Methyl, Ethyl, 1-Methylethyl, 1-Methylpropyl, 2-Methylpropyl, 1,1-Dimethylethyl und 1,1-Dimethylpropyl;
   unverzweigtes oder verzweigtes C₃-C₂₅-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl, 10-Undecenyl, 9-Tetradecenyl, 9-Hexadecenyl, 6-Octadecenyl, 9-Octadecenyl, 11-Octadecenyl, 9,12-Octadecadienyl, 11-Eicosenyl, 13-Eicosenyl und 13-Docosenyl;
   insbesondere C₃-C₁₅-Alkenyl, beispielsweise C₃-C₆-Alkenyl wie 2-Propenyl, 2-Butenyl, 3-Butenyl, 1-Methyl-2-propenyl, 2-Methyl-2-propenyl und 1-Methyl-2-butenyl;
   C₃-C₂₅-Alkinyl, insbesondere C₃-C₁₅-Alkinyl, besonders bevorzugt C₃-C₈-Alkinyl, z.B. Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
   C₃-C₈-Cycloalkyl wie Cyclopropyl, Cyclobutyl, Cyclopentyl, Cyclohexyl, Cycloheptyl, Cyclooctyl, insbesondere Cyclopropyl und Cyclohexyl;
   wobei die vorstehend genannten organischen Gruppen ein bis fünf Halogenatome wie Fluor, Chlor, Brom und Iod, vorzugsweise Fluor, Chlor und Brom, insbesondere Fluor und Chlor,
   und/oder einen bis drei der folgenden Reste tragen können:
   Cyano;
   C₃-C₈-Cycloalkyl wie Cyclopropyl, Cyclobutyl, Cyclopentyl, Cyclohexyl, Cycloheptyl, Cyclooctyl, insbesondere Cyclopropyl und Cyclohexyl;
   C₁-C₄-Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere Methoxy und Ethoxy;
   C₁-C₄-Halogenalkoxy wie Chlormethoxy, Dichlormethoxy, Trichlormethoxy, Fluormethoxy, Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere 2,2,2-Trifluorethyloxy und 2-Chlor-2,2-difluorethoxy;
   C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
   C₁-C₄-Halogenalkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trichlormethylthio;
   Phenyl, Phenylthio, Phenoxy, wobei die Phenylreste ihrerseits eine bis fünf Halogenatome wie Fluor, Chlor, Brom und Iod, insbesondere Fluor und Chlor,
   und/oder eine bis drei der folgenden Gruppen tragen können:
   Cyano oder Nitro;
   C₁-C₄-Alkyl, wie vorstehend genannt, insbesondere Methyl, Ethyl, Propyl, 1-Methylpropyl und 1,1-Dimethylpropyl;
   C₁-C₄-Alkoxy, wie vorstehend genannt, insbesondere Methoxy und Ethoxy;
   C₁-C₄-Halogenalkyl, wie vorstehend genannt, insbesondere Trifluormethyl;
   C₁-C₄-Halogenalkoxy, wie vorstehend genannt, insbesondere Trifluormethoxy;
R²
   Wasserstoff, Hydroxy;
   C₁-C₈-Alkoxy, z.B. Methoxy, Ethoxy, n-Propoxy, 2-Methylethoxy, n-Butoxy, 1-Methylpropoxy, 2-Methylpropoxy und 1,1-Dimethylethoxy, insbesondere C₁-C₄-Alkoxy wie Methoxy, Ethoxy, Propoxy, Butoxy;
   C₃-C₈-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 3-Methyl-2-butenyloxy, 1-Methyl-2-butenyloxy, 2-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-4-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-4-butenyl, 1-Ethyl-2-butenyl, 1-Ethyl-3-butenyl, 2-Ethyl-2-butenyloxy, 2-Ethyl-3-butenyloxy, 1,1,2-Trimethyl-2-propenyloxy, 1-Ethyl-1-methyl-2-propenyloxy und Ethyl-2-methyl-2-propenyloxy, insbesondere 2-Propenyloxy;
   Di-C₁-C₄-Alkylamino, C₁-C₂₅-Alkyl, C₃-C₂₅-Alkenyl und -Alkinyl sowie C₃-C₈-Cycloalkyl, jeweils wie vorstehend für R¹ im allgemeinen und im besonderen genannt,
   wobei die genannten organischen Gruppen ein bis fünf Halogenatome wie Fluor, Chlor, Brom und Iod, vorzugsweise Fluor, Chlor und Brom, insbesondere Fluor und Chlor,
   und/oder einen bis drei der folgenden Reste tragen können:
   Cyano,
   C₃-C₈-Cycloalkyl, C₁-C₄-Alkoxy, C₁-C₄-Halogenalkoxy, C₁-C₄-Alkylthio, C1-C4-Halogenalkylthio und Di-C₁-C₄-Alkylamino, jeweils wie vorstehend im einzelnen angegeben,
   Phenyl, Phenylthio, Phenoxy, wobei die Phenylreste ihrerseits eine bis fünf Halogenatome wie Fluor, Chlor, Brom und Iod, insbesondere Fluor und Chlor,
   und/oder eine bis drei der folgenden Gruppen tragen können:
   Cyano oder Nitro,
   C₁-C₄-Alkyl, C₁-C₄-Alkoxy, C₁-C₄-Halogenalkyl und C₁-C₄-Halogenalkoxy, jeweils wie vorstehend genannt;
   R² ferner substituiertes C₁-C₈-Alkoxy, C₃-C₈-Alkenyloxy, Di-C₁-C₄-Alkylamino, C₁-C₂₅-Alkyl, C₃-C₂₅-Alkenyl, C₃-C₂₅-Alkinyl oder C₃-C₈-Cycloalkyl, wobei diese Gruppen durch ein bis fünf Halogenatome und/oder ein bis zwei 5- bis 6-gliedrige heterocyclische, aliphatische oder aromatische Reste, enthaltend ein bis drei Heteroatome, ausgewählt aus der Gruppe Sauerstoff, Schwefel oder Stickstoff, substituiert sind; beispielsweise seien folgende heterocyclische Reste genannt: 2-Tetrahydrofuranyl, 3-Tetrahydrofuranyl, 2-Tetrahydrothienyl, 3-Tetrahydrothienyl, 2-Tetrahydropyranyl, 3-Tetrahydropyranyl, 4-Tetrahydropyranyl, 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazolyl, Thia-2,4-diazolyl, Thia-3,4-diazolyl und Triazolyl;
   wobei diese heterocyclischen Reste einen oder zwei der folgenden Substituenten tragen können:
   Halogen wie Fluor, Chlor, Brom und Iod, insbesondere Fluor und Chlor, C₁-C₃-Alkyl oder C₁-C₃-Alkoxy, wie vorstehend genannt;
   R¹ und R² gemeinsam eine C₄-C₇-Alkylenkette, welche durch Sauerstoff, Schwefel oder N-Methyl unterbrochen sein kann wie -(CH₂)₃-, -(CH₂)₄-, -(CH₂)₅-, -(CH₂)₆-, -CH₂-O-CH₂-, -CH₂-CH₂-O-CH₂-CH₂-, -CH₂-S-CH₂-, -CH₂-CH₂-S-CH₂-CH₂-, -CH₂-N(CH₃)-CH₂-CH₂-, insbesondere -(CH₂)₅- und -CH₂-CH₂-O-CH₂-CH₂-;
R³ bis R⁶
   Wasserstoff;
   Halogen wie Fluor, Chlor, Brom und Iod, insbesondere Fluor und Chlor;
   Cyano oder Nitro;
   C₁-C₄-Alkyl, C₁-C₄-Alkoxy, C₁-C₄-Halogenalkyl, C₁-C₄-Halogenalkoxy, C₁-C₄-Alkylthio oder C₁-C₄-Halogenalkylthio, jeweils wie vorstehend für R¹ im einzelnen genannt.

Als Salze der Verbindungen I kommen landwirtschaftlich brauchbare Salze, beispielsweise Alkalimetallsalze, insbesondere das Kalium- oder Natriumsalz, Erdalkalimetallsalze, insbesondere das Calcium-, Magnesium- oder Bariumsalz, Mangan-, Kupfer-, Zink- oder Eisensalze sowie Ammonium-, Phosphonium-, Tetraalkylammoniumsalze, Benzyltrialkylammoniumsalze, Trialkylsulfoniumsalze oder Trialkylsulfoxoniumsalze in Betracht.

Beispiele für besonders bevorzugte Verbindungen der allgemeinen Formel I sind in der folgenden Tabelle zusammengestellt:

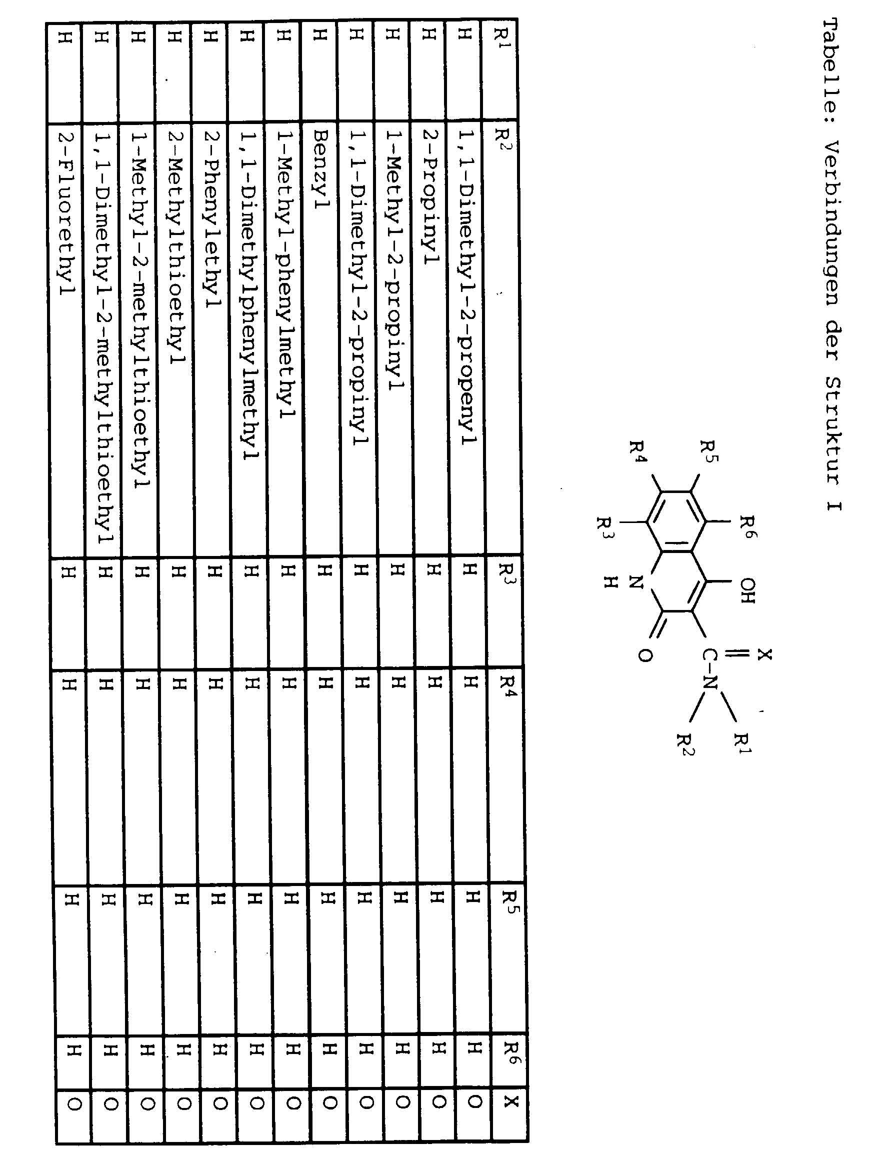

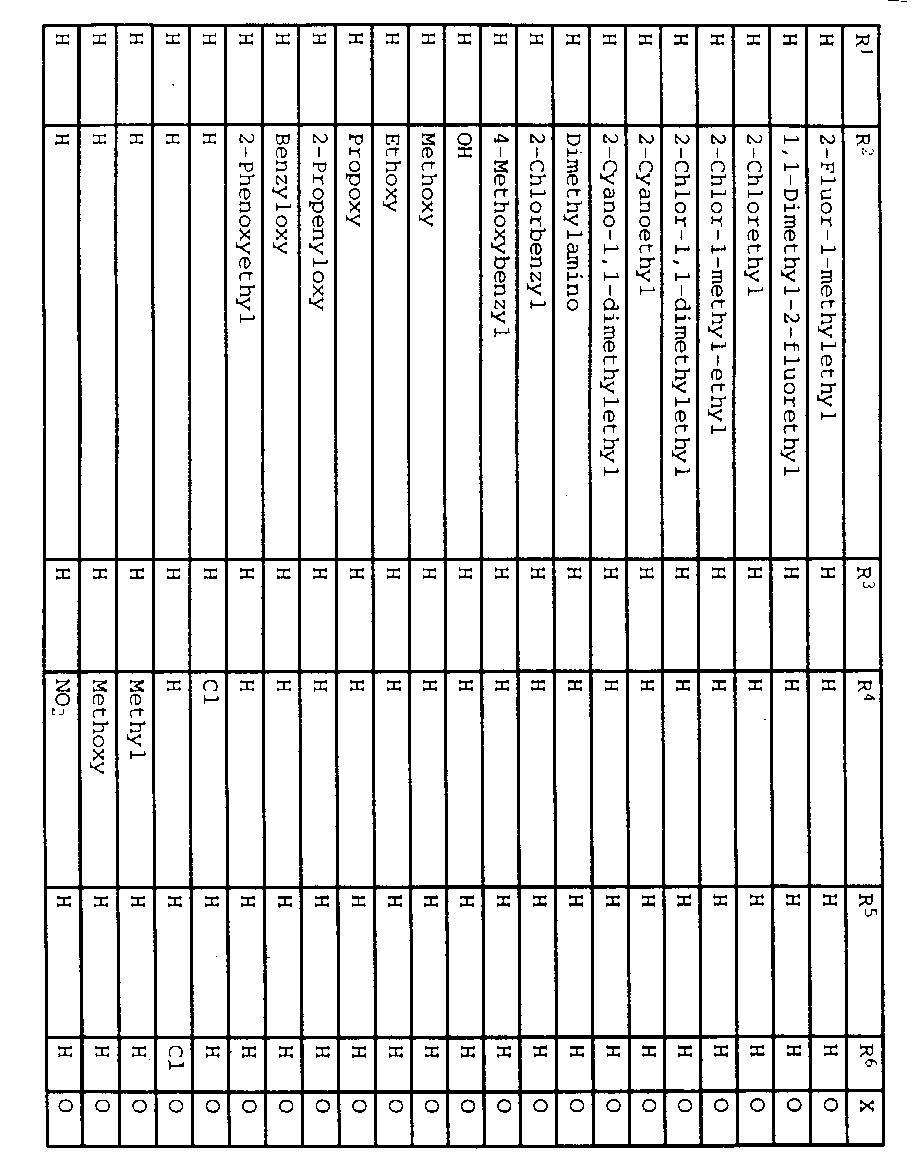

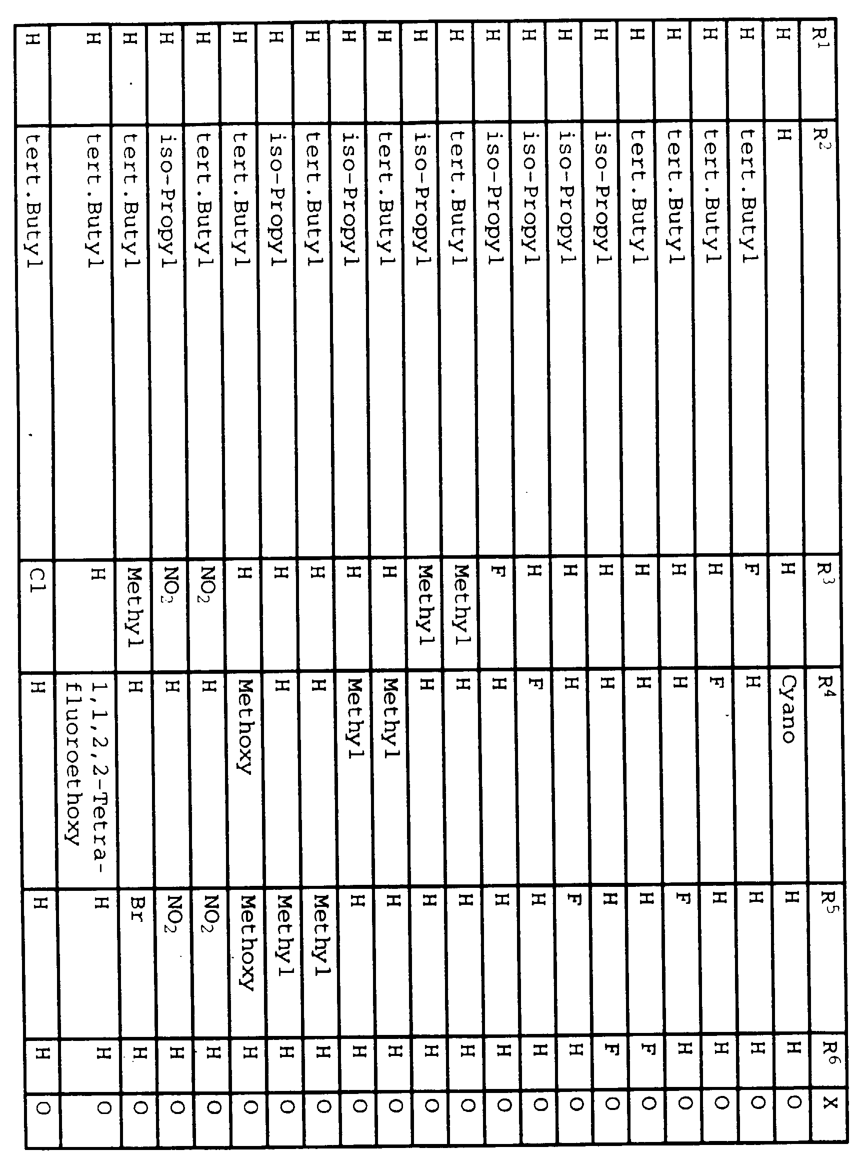

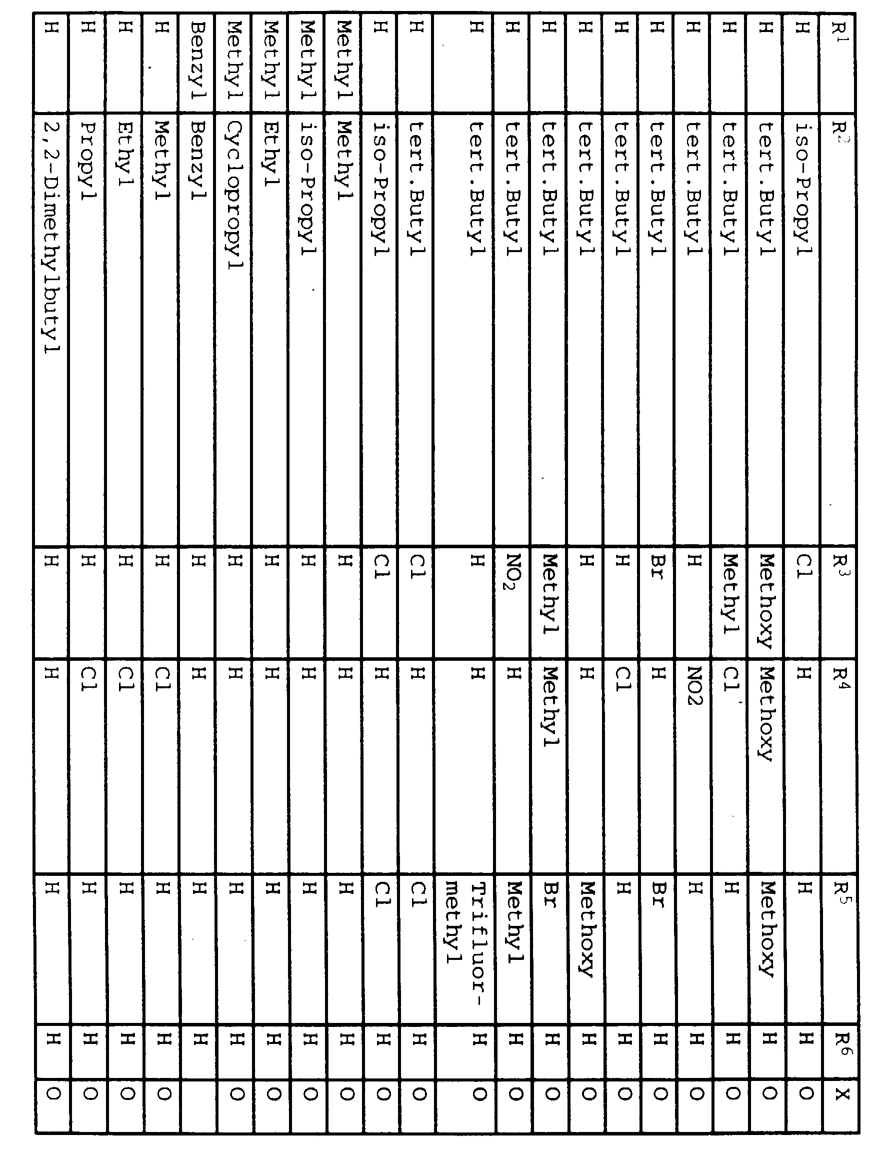

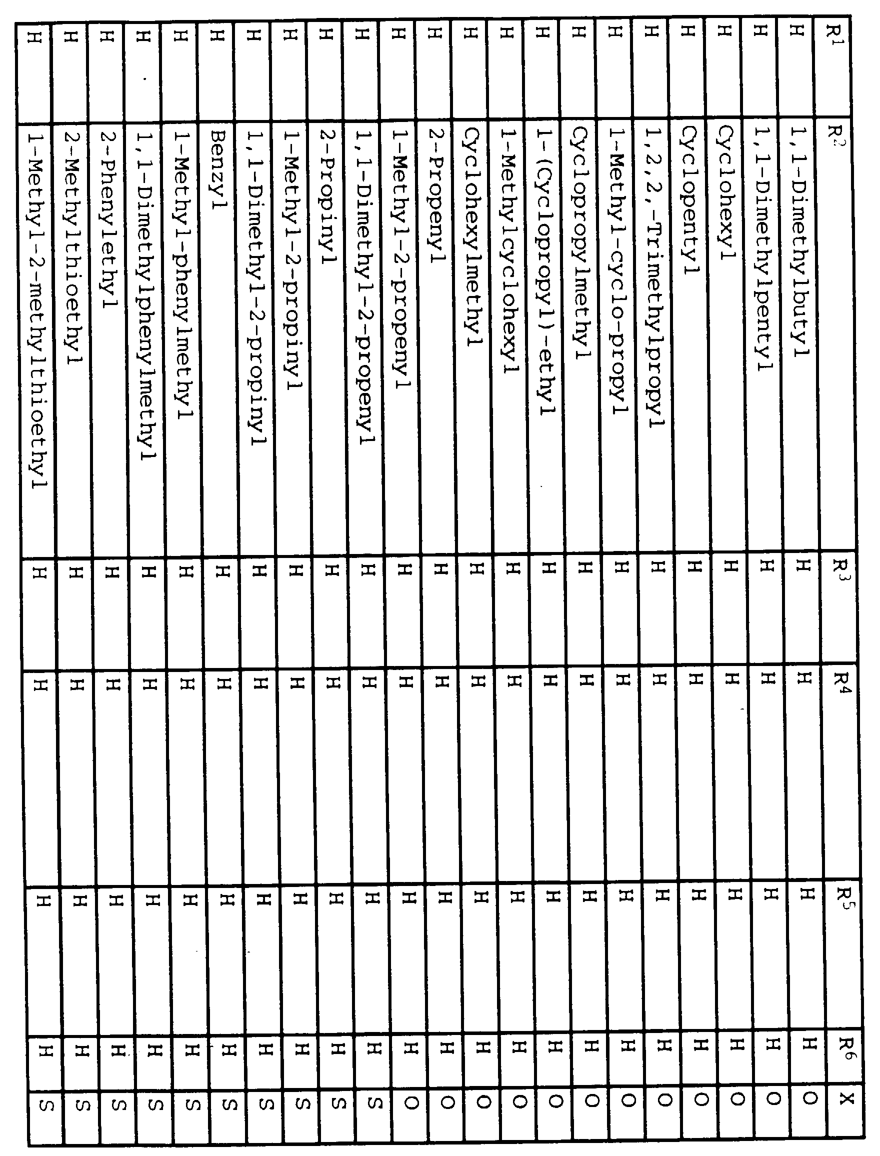

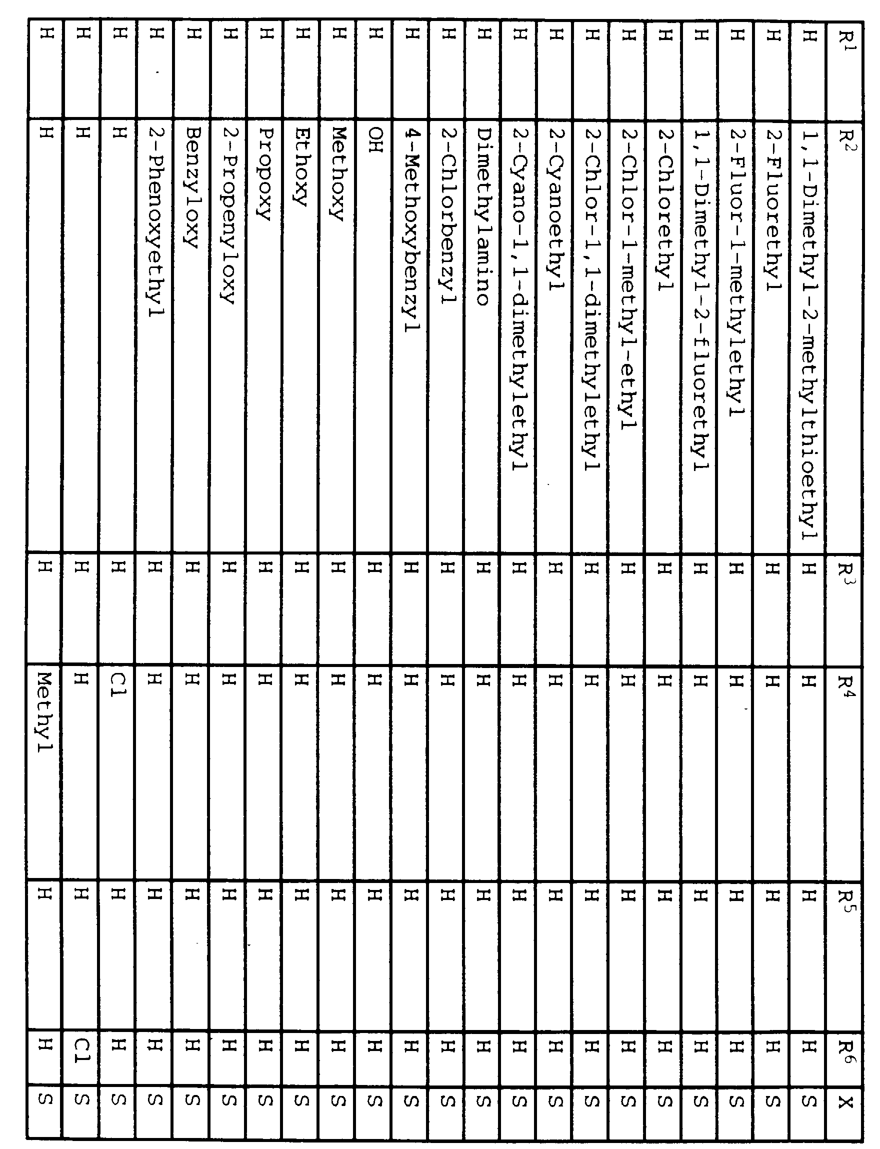

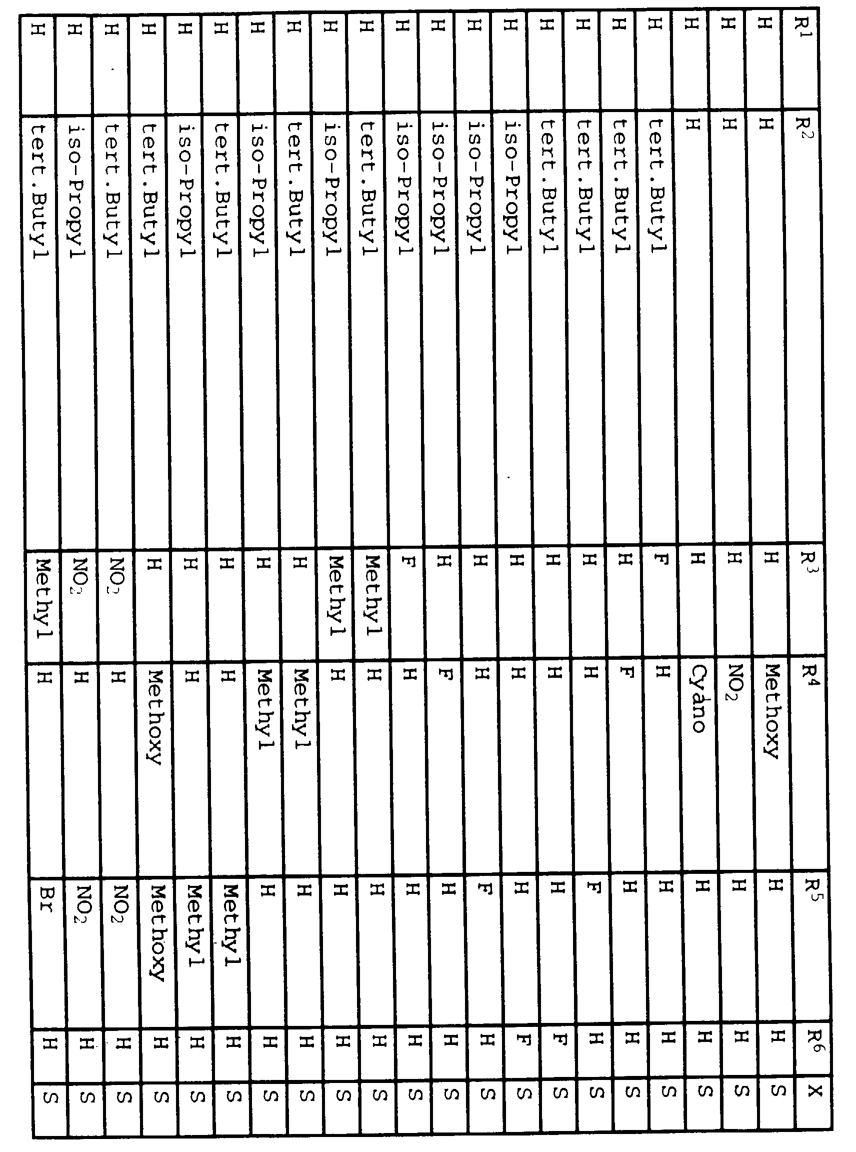

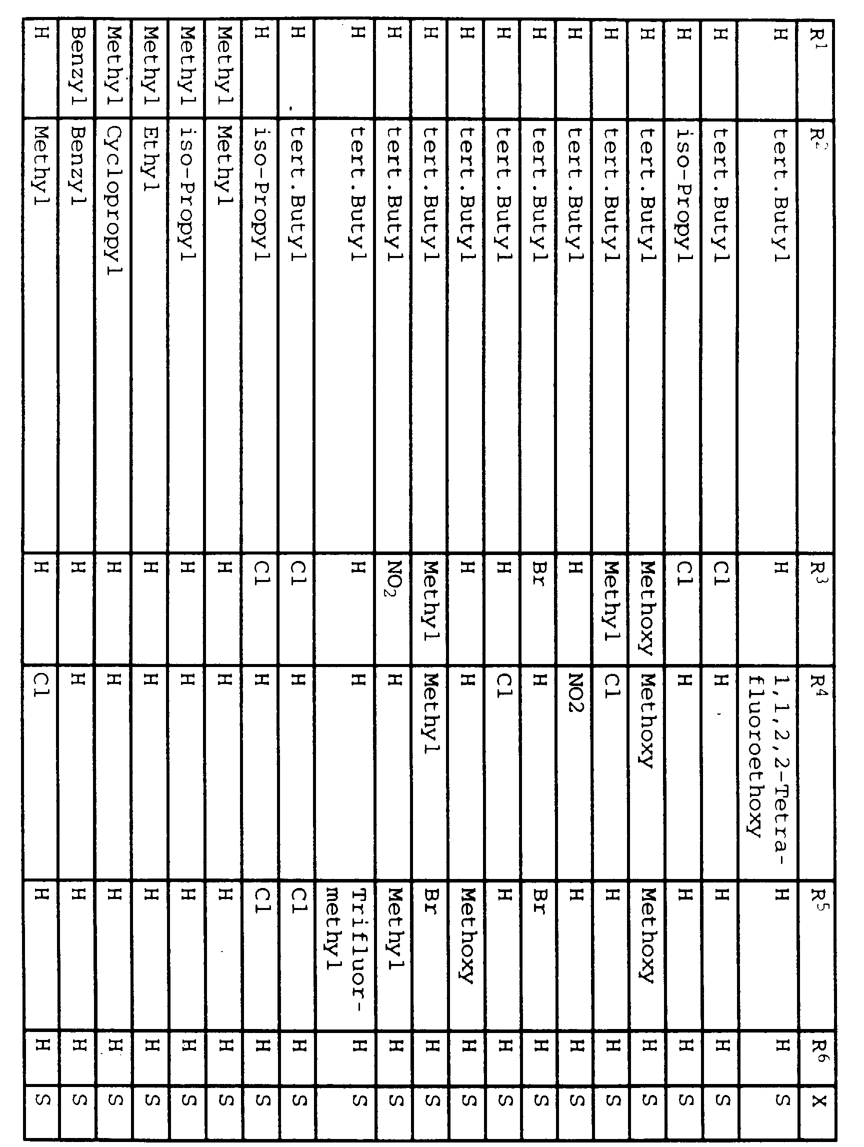

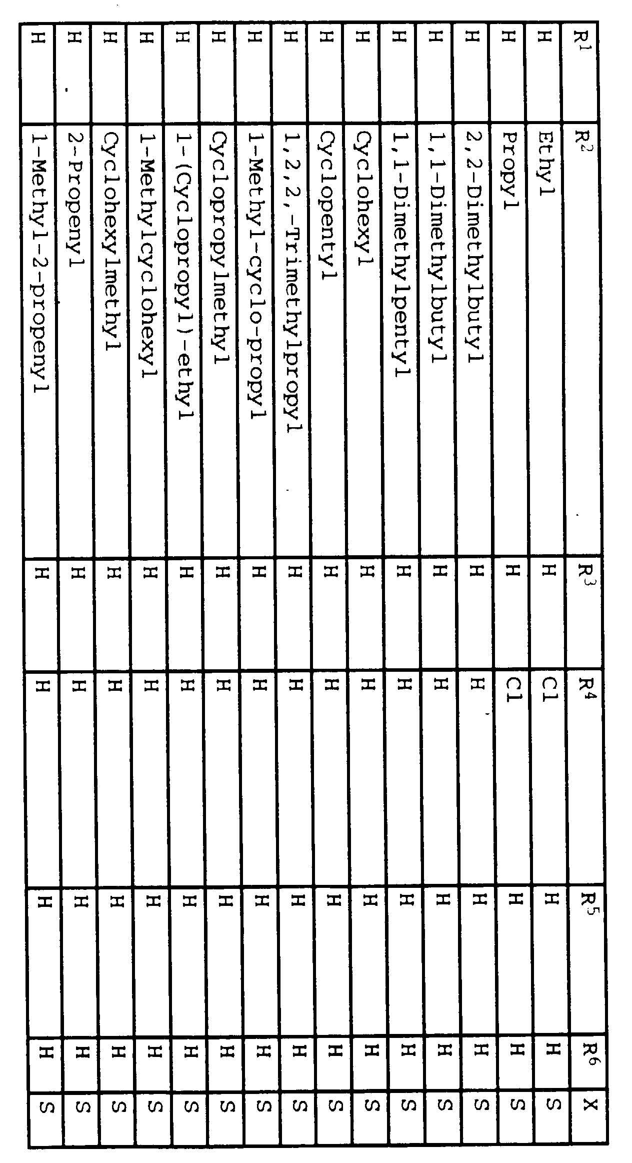

Die Verbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, vernetzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, LigninSulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (nach NMR-Spektrum) eingesetzt.

Die erfindungsgemäßen Verbindungen I können beispielsweise wie folgt formuliert werden:
- I.: Man vermischt 90 Gew.-Teilen der Verbindung Nr. 1.01 mit 10 Gew.-Teilen N-Methyl-α-pyrrolidon und erhält eine Lösung, die zur Anwendung in Form kleinster Tropfen geeignet ist;
- II.: 20 Gew.-Teile der Verbindung Nr. 1.05 werden in einer Mischung gelöst, die aus 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl besteht. Durch Ausgießen und feines Verteilen der Lösung in 100.000 Gew.-Teilen Wasser erhält man eine wäßrige Dispersion, die 0,02 Gew.-% des Wirkstoffs enthält.
- III.: 20 Gew.-Teile der Verbindung Nr. 1.01 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
- IV.: 20 Gew.-Teile des Wirkstoffs Nr. 1.05 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
- V.: 20 Gew.-Teile des Wirkstoffs Nr. 1.01 werden mit 3 Gew.-Teilen des Natriumsalzes der Diisobutylnaphtalin-α-sulfonsäure, 1 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.
- VI.: 3 Gew.-Teilen des Wirkstoffs Nr. 1.01 werden mit 97 Gew.-Teilen feinteiligem Kaolin vermischt. Man erhält auf diese Weise ein Stäubemittel, das 3 Gew.-% des Wirkstoffs enthält.
- VII.: 30 Gew.-Teilen des Wirkstoffs Nr. 1.01 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese weise eine Aufbereitung des Wirkstoffs mit guter Haftfähigkeit.
- VIII.: 20 Gew.-Teile des Wirkstoffs Nr. 1.01 werden mit 2 Gew.-Teilen Calciumsalz der Dodecylbenzolsulfonsäure, 8 Gew.-Teilen Fettalkohol-polyglykolether, 2 GEw.-Teilen Natriumsalz eines Phenolsulfonsäureharnstoff-formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender-unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

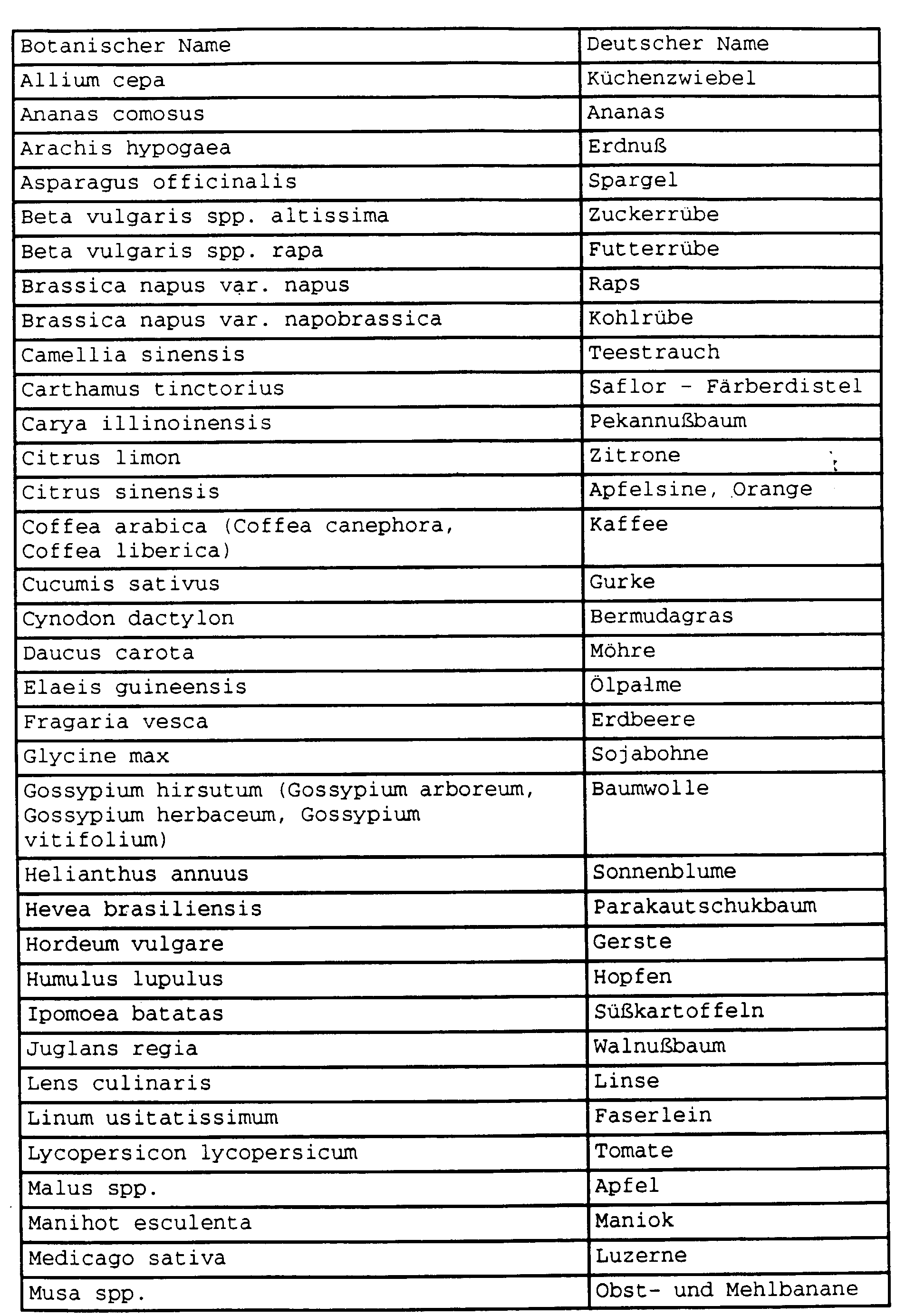

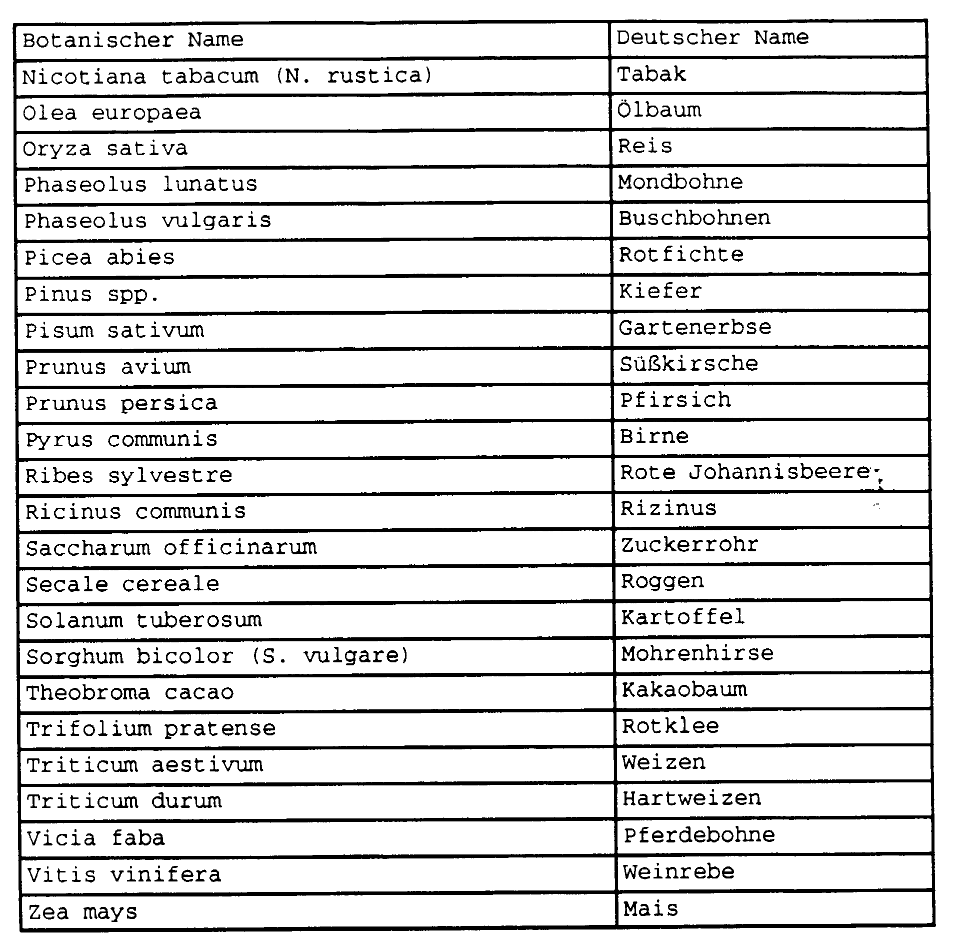

Zur Verbreiterung des Wirkungsspektrums und zur Erzielung synergistischer Effekte können die Chinolin-3-carbonsäureamide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Beispiel 1

### Herstellung von 4-Hydroxy-2-oxo-chinolin-3-carbonsäuretert.-butylamid

8,76 g (0,04 mol) 4-Hydroxy-2-oxo-chinolin-3-carbonsäuremethylester wurden mit 3,7 g (0,05 mol) tert.-Butylamin in 60 ml Ethanol in einem Miniautoklaven 8 Stunden bei 150°C unter Eigendruck gerührt. Nach dem Abkühlen wurden die Kristalle abgesaugt, mit Petrolether gewaschen und getrocknet.

Fp.: 230-231°C; Ausbeute: 7,7 g, 74 % der Theorie.

In entsprechender Weise wurden unter Abwandlung der Ausgangsstoffe weitere Verbindungen I hergestellt. Die so erhaltenen Verbindungen sind in der nachstehenden Tabelle 1 mit physikalischen Angaben aufgeführt.

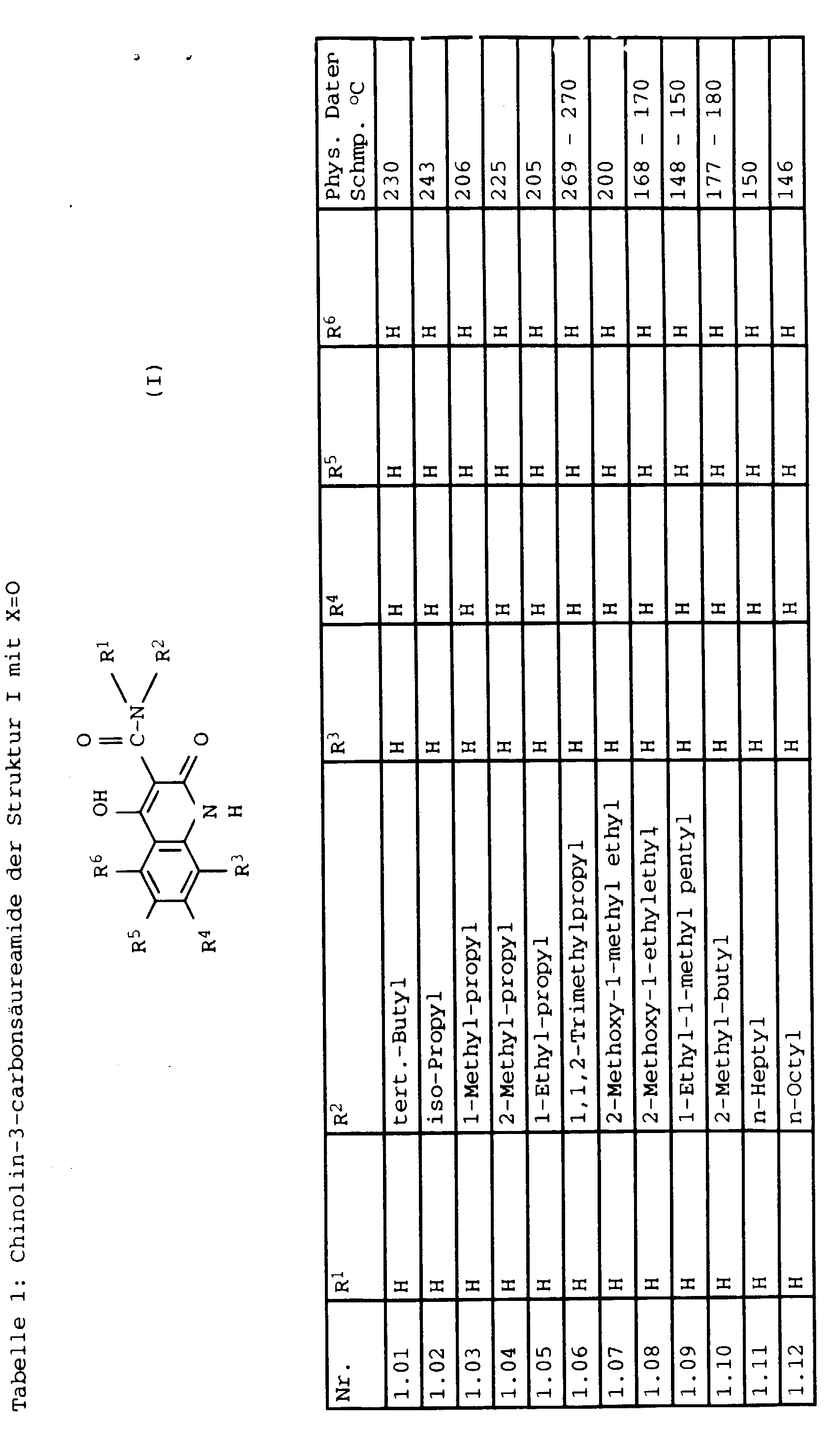

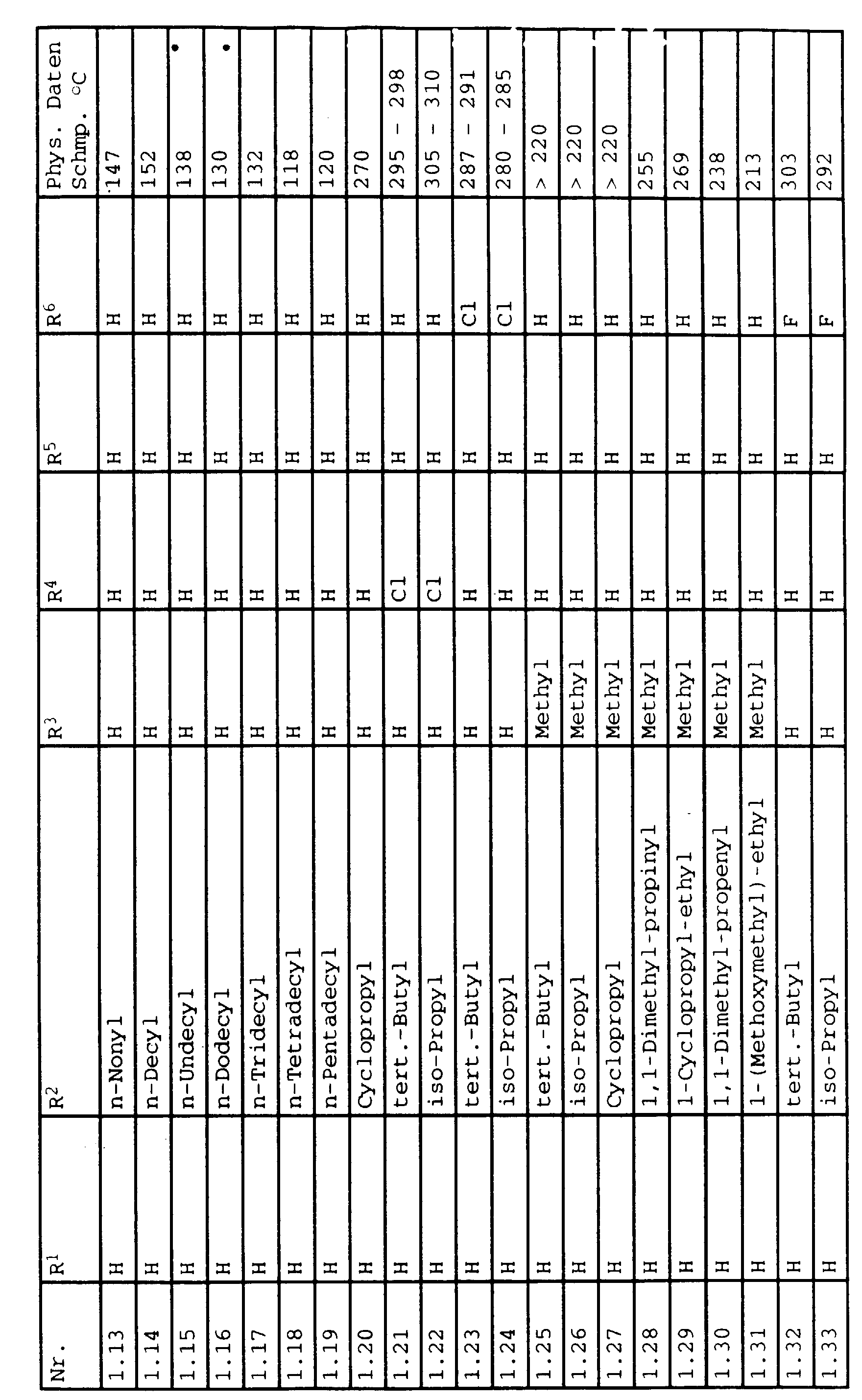

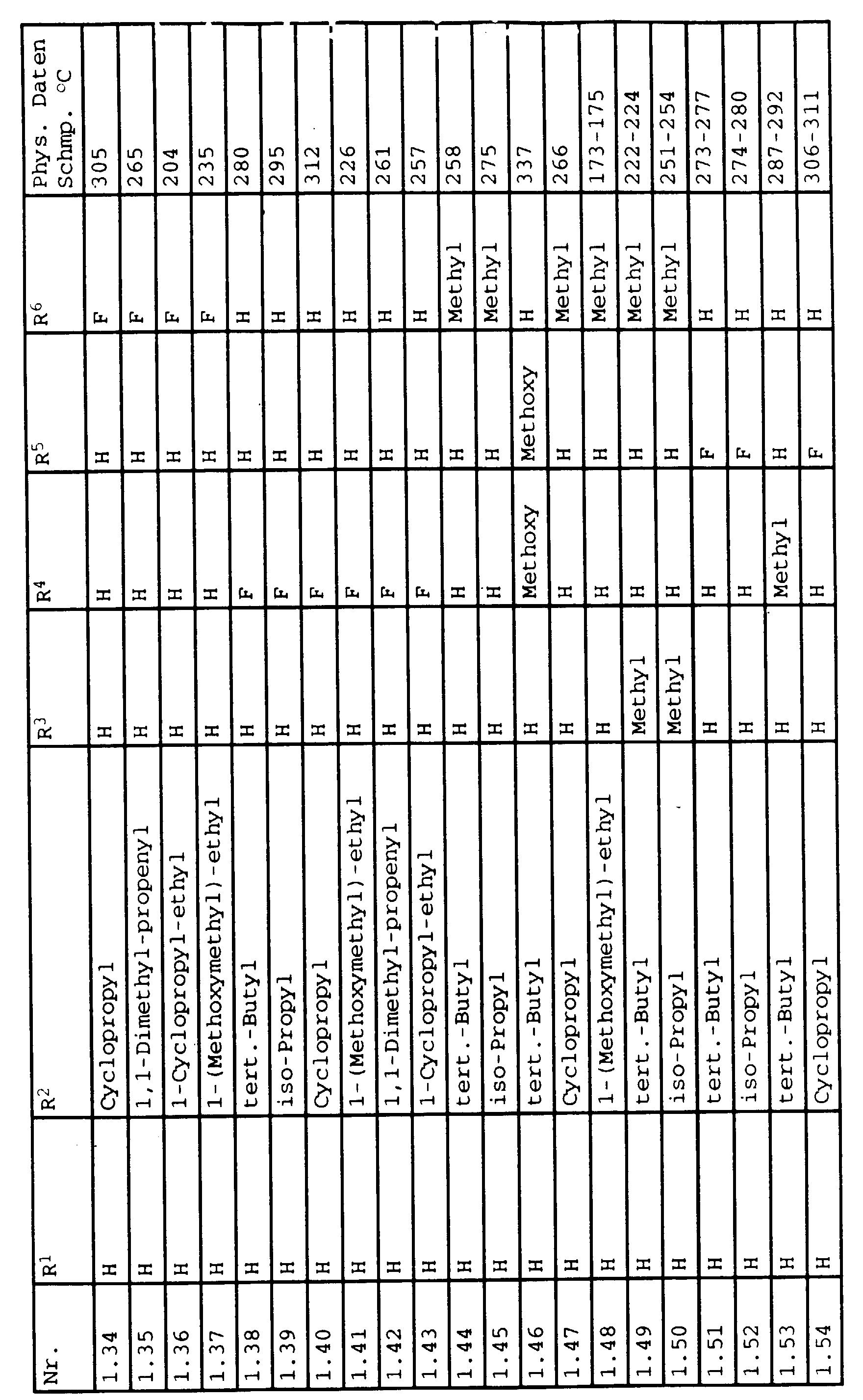

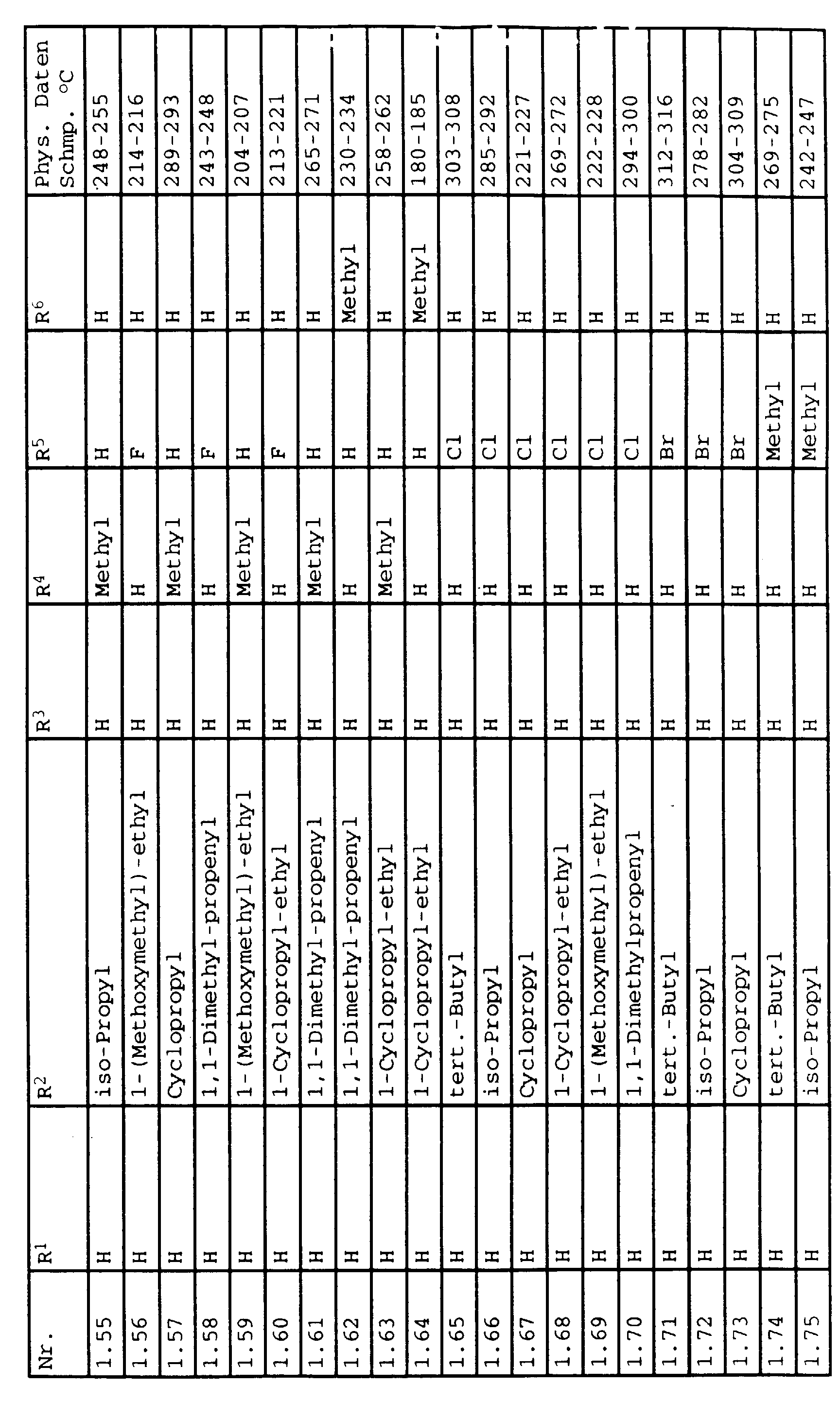

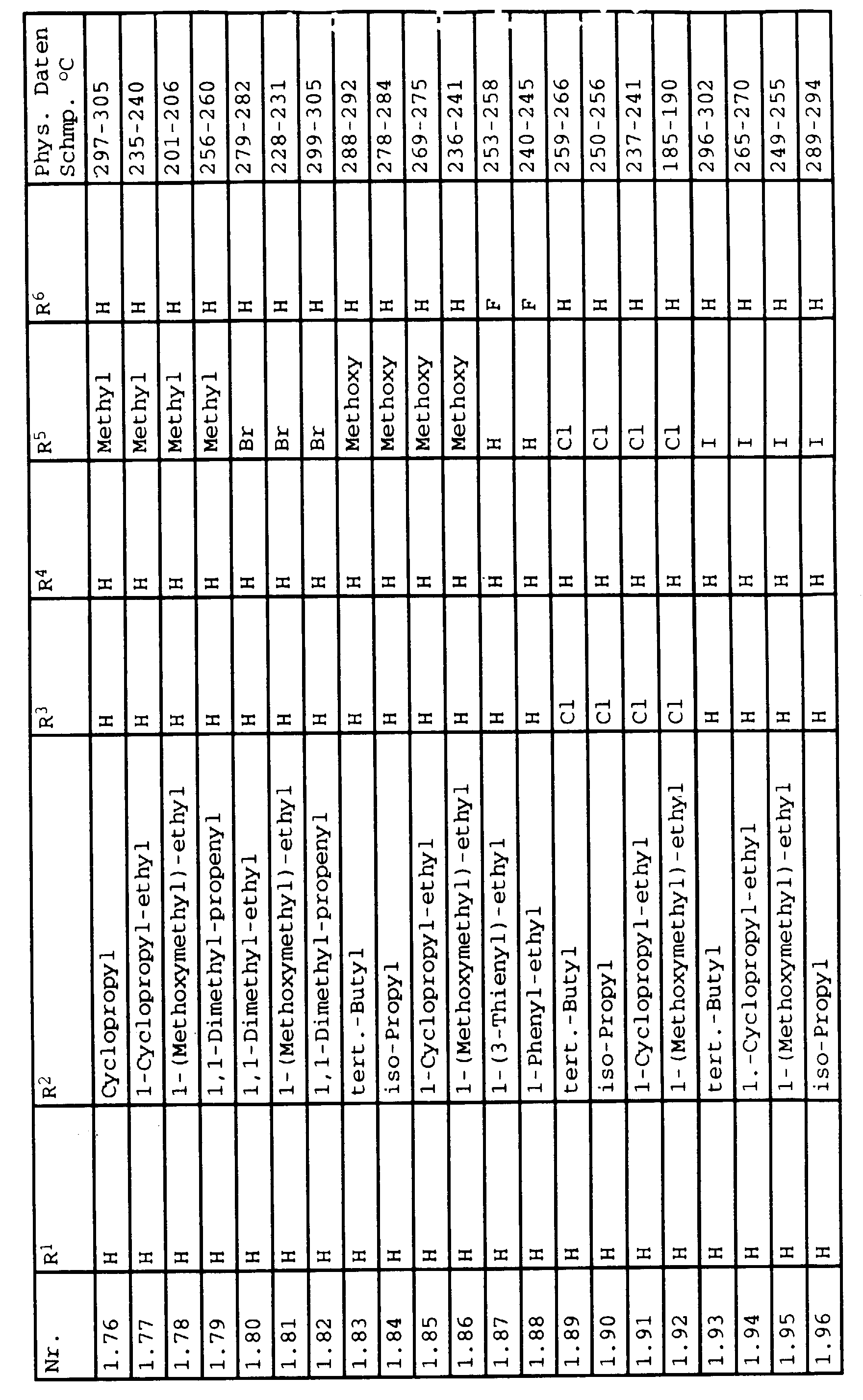

### Beispiel 2

### Herstellung von 5-Fluor-4-hydroxy-2-oxo-chinolin-3-carbonsäure-tert.-butylamid, Na-Salz

6,0 g (0,023 mol) 5-Fluor-4-hydroxy-2-oxo-chinolin-3-carbonsäure-tert.-butylamid werden in Methanol suspendiert und mit einer Lösung von 1,25 g Natriummethylat in Methanol versetzt. Die Lösung wird 1 Stunde bei Raumtemperatur gerührt, anschließend eingeengt und der Rückstand getrocknet.

Fp.: > 399°C; Ausbeute: 6,4 g, 98 % der Theorie

In entsprechender Weise wurden unter Abwandlung der Ausgangsstoffe weitere Salze von Verbindungen I hergestellt. Die so erhaltenen Salze sind in der nachstehenden Tabelle 2 mit physikalischen Angaben aufgeführt.

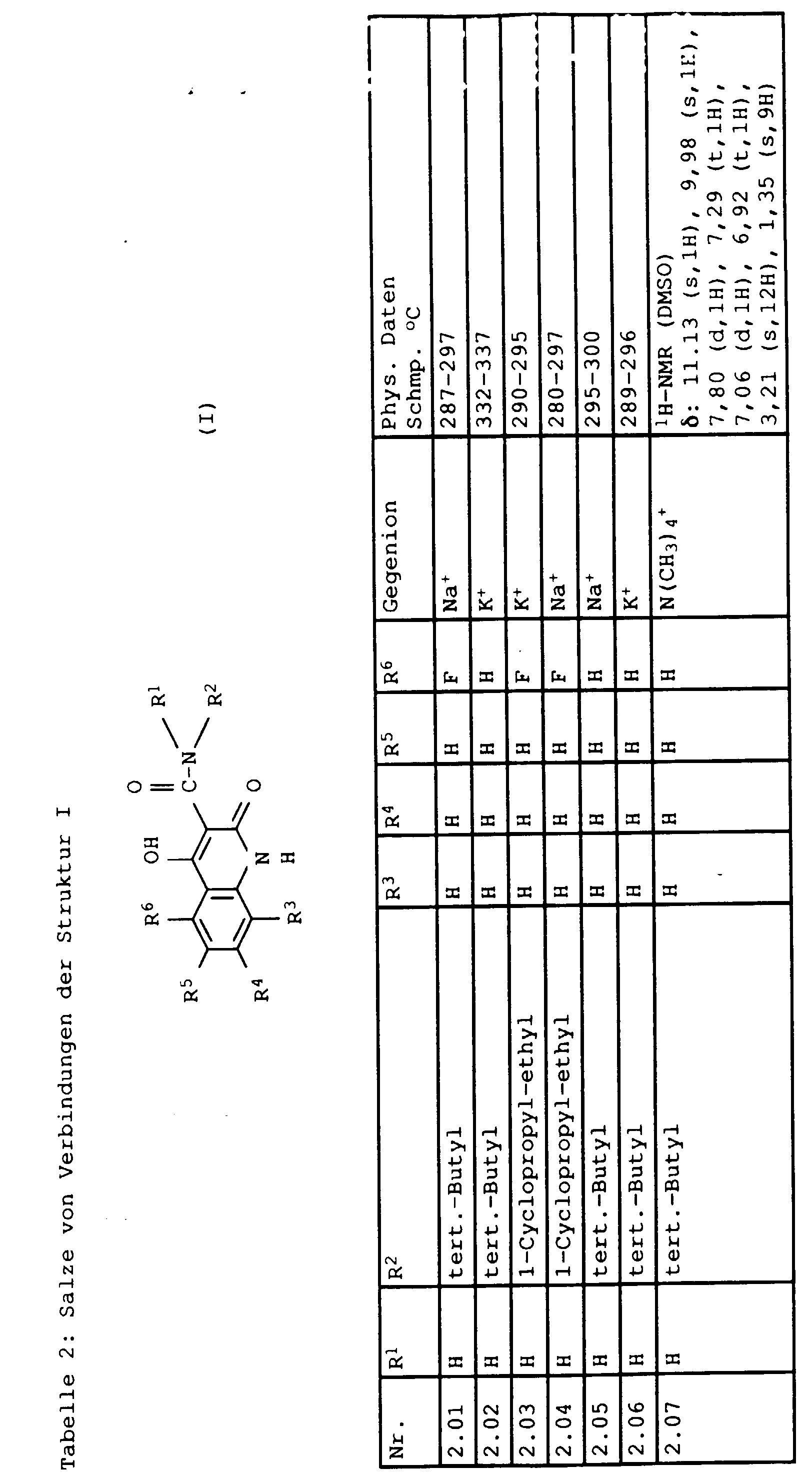

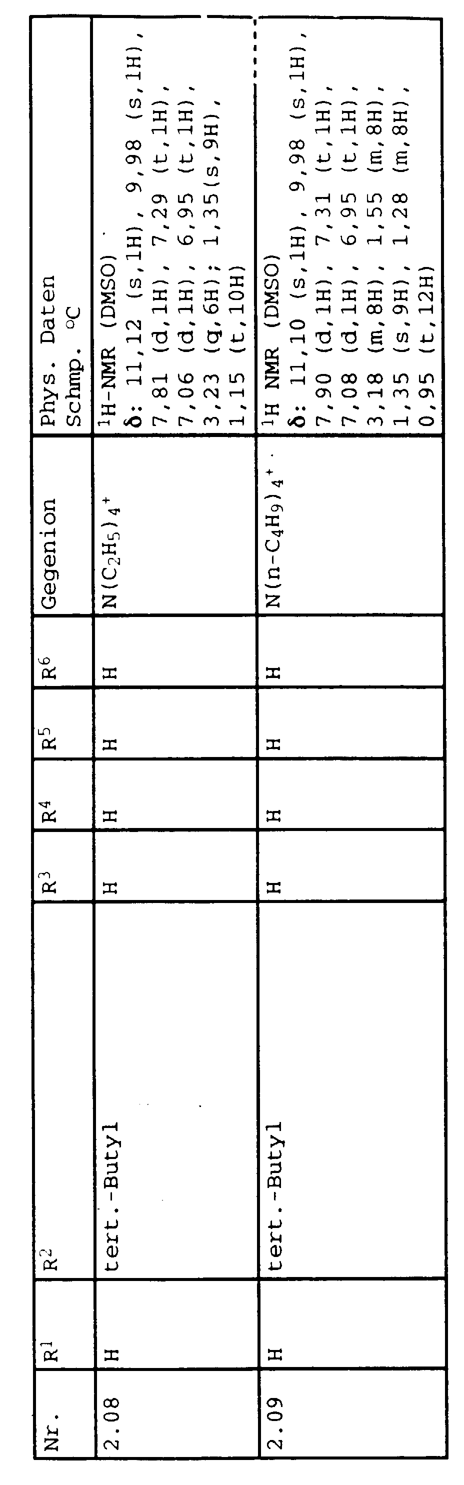

### Beispiel 3

Die Herstellung von Chinolin-3-carbonsäureestern als Zwischenprodukte für die Synthese der entsprechenden Amide erfolgte wie in Australian Journal of Chem. Vol. 7, S. 348 u. 368 (1954) beschrieben.

Ausgewählte Beispiele sind in Tabelle 3 zusammengestellt.

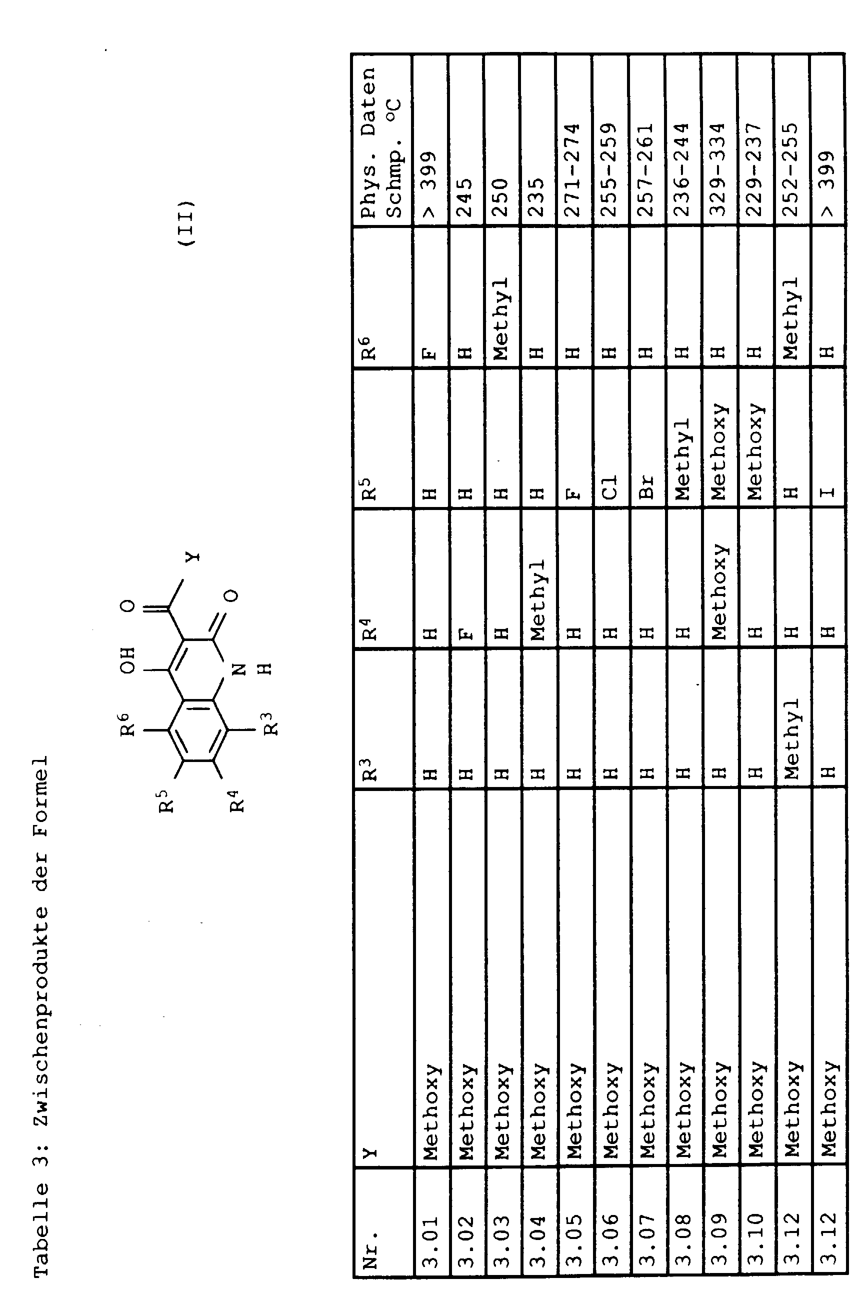

### Anwendungsbeispiele

Die herbizide Wirkung der Verbindungen I ließ sich durch Gewächshausversuche zeigen:

Als Kulturgefäße dienten Plastikblumentöpfe mit lehmigem Sand mit etwa 3 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 3 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:
- Lateinischer Name: Deutscher Name
- Echinochloa crus-galli: Hühnerhirse
- Centaurea cyanus: Kornblume

Mit 3 kg/ha a.S. im Nachauflaufverfahren eingesetzt, erzielt man mit den Beispielen 1.01 und 1.05 eine sehr gute herbizide Wirkung.

## Patentansprüche

1. Chinolin-3-carbonsäureamide der allgemeinen Formel I, 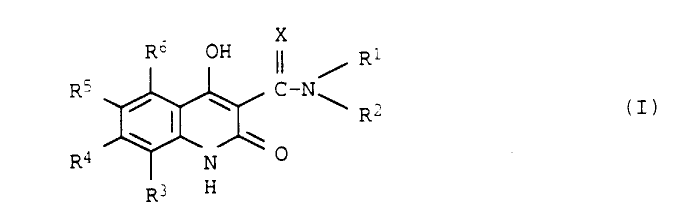 in der die Substituenten folgende Bedeutung haben:
R¹ Wasserstoff, C₁-C₂₅-Alkyl, C₃-C₂₅-Alkenyl, C₃-C₂₅-Alkinyl, C₃-C₈-Cycloalkyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
R² Wasserstoff, Hydroxyl, C₁-C₈-Alkoxy, C₃-C₈-Alkenyloxy Di-C₁-C₄-Alkylamino, C₁-C₂₅-Alkyl, C₃-C₂₅-Alkenyl, C₃-C₂₅-Alkinyl, C₃-C₈-Cycloalkyl, wobei die genannten organischen Reste ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
R² ferner substituiertes C₁-C₈-Alkoxy, C₃-C₈-Alkenyloxy, Di-C₁-C₄-Alkylamino, C₁-C₂₅-Alkyl, C₃-C₂₅-Alkenyl, C₃-C₂₅-Alkinyl oder C₃-C₈-Cycloalkyl, wobei diese Gruppen durch ein bis fünf Halogenatome und/oder ein bis zwei 5- bis 6-gliedrige heterocyclische, aliphatische oder aromatische Reste, enthaltend ein bis drei Heteroatome, ausgewählt aus der Gruppe Sauerstoff, Schwefel oder Stickstoff, substituiert sind, die ein oder zwei der folgenden Substituenten tragen können: Halogen, C₁-C₃-Alkyl oder C₁-C₃-Alkoxy;
oder
R¹, R² gemeinsam eine Alkylenkette mit 4 bis 7 Gliedern, welche durch Sauerstoff, Schwefel oder N-Methyl unterbrochen sein kann;
R³-R⁶ Wasserstoff, C₁-C₄-Alkyl, C₁-C₄-Alkoxy, C₁-C₄-Halogenalkyl, C₁-C₄-Halogenalkoxy, C₁-C₄-Alkylthio, C₁-C₄-Halogenalkylthio, Halogen, Cyano oder Nitro;
X Sauerstoff oder Schwefel;
und deren umweltverträgliche Salze,
mit der Maßgabe, daß R² nicht Wasserstoff, C₁-C₃-Alkyl, n-Butyl, 3-Methylbutyl, Cyclohexyl, Hexyl, Heptyl, Octyl, 2-Chlorbenzyl, 3-(Dimethylamino)propyl, 3-(Diethylamino)propyl, 2-Morpholinoethyl und 2-(3,4-Dimethoxyphenyl)ethyl bedeutet, wenn R¹ und R³ bis R⁶ Wasserstoff und X Sauerstoff bedeuten und R² nicht Benzyl bedeutet, wenn R¹ Methyl, R³ bis R⁶ Wasserstoff und X Sauerstoff bedeutet, sowie R¹ und R² nicht gemeinsam für Morpholino stehen, wenn R³ bis R⁶ Wasserstoff und X Sauerstoff bedeuten; ausgenommen ferner die Verbindungen 7-Chlor-2,4-dioxo-3-(1,2,3,4-tetrahydrochinolin)hydroxamsäure und 7-Chlor-5-iod-2,4-dioxo-3-(1,2,3,4-tetrahydrochinolin)hydroxamsäure.

2. Chinolin-3-carbonsäureamide der Formel I gemäß Anspruch 1, in der X Sauerstoff oder Schwefel, R¹ Wasserstoff und
R² verzweigtes oder unverzweigtes C₁-C₁₅-Alkyl, C₃-C₁₅-Alkenyl, C₃-C₁₅-Alkinyl, C₁-C₅-Alkoxy, C₃-C₆-Cycloalkyl, wobei diese genannten Reste durch Halogen, C₁-C₃-Alkoxy, C₁-C₃-Halogenalkoxy oder gegebenenfalls substituiertes Phenyl substituiert sein können;
bedeuten.

3. Verfahren zur Herstellung der Chinolin-3-carbonsäureamide I gemäß Anspruch 1, dadurch gekennzeichnet, daß man ein Chinolin-3-carbonsäurederivat der Formel II 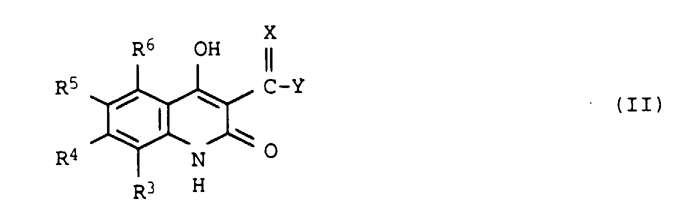 in der X und R³ bis R⁶ die in Anspruch 1 angegebenen Bedeutungen haben und Y Hydroxyl, Halogen oder eine Alkoxygruppe bedeuten, in an sich bekannter Weise mit einem substituierten Amin der Formel III 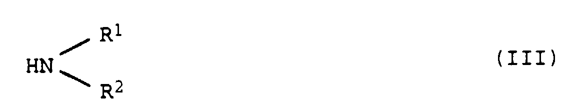 umsetzt.

4. Verfahren zur Herstellung der Chinolin-3-carbonsäureamide der Formel I gemäß Anspruch 1, in der X für Sauerstoff und R1 für Wasserstoff stehen, dadurch gekennzeichnet, daß man ein 2,4-Dihydroxychinolin der Formel IV 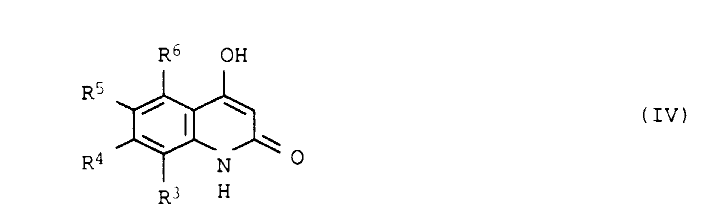 mit einem Isocyanat der Formel V
O=C=N-R² (V)
umsetzt.

5. Herbizide Mittel, enthaltend ein Chinolin-3-carbonsäureamid der Formel I gemäß Anspruch 1 und inerte Zusatzstoffe.

6. Verfahren zu Bekämpfung unerwünschten Pflanzenwuchses, dadurch gekennzeichnet, daß man die unerwünschten Pflanzen und/oder ihren Lebensraum mit einer herbizid wirksamen Menge eines Chinolin-3-carbonsäureamids der Formel I 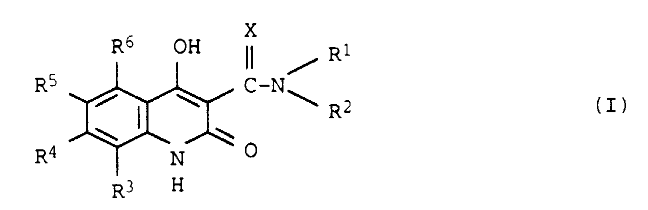 in der die Substituenten folgende Bedeutung haben:
R¹ Wasserstoff, C₁-C₂₅-Alkyl, C₃-C₂₅-Alkenyl, C₃-C₂₅-Alkinyl, C₃-C₈-Cycloalkyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
R² Wasserstoff, Hydroxyl, C₁-C₈-Alkoxy, C₃-C₈-Alkenyloxy, Di-C₁-C₄-Alkylamino, C₁-C₂₅-Alkyl, C₃-C₂₅-Alkenyl, C₃-C₂₅-Alkinyl, C₃-C₈-Cycloalkyl, wobei die genanten organischen Reste ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
R² ferner substituiertes C₁-C₈-Alkoxy, C₃-C₈-Alkenyloxy, Di-C₁-C₄-Alkylamino, C₁-C₂₅-Alkyl, C₃-C₂₅-Alkenyl, C₃-C₂₅-Alkinyl oder C₃-C₈-Cycloalkyl, wobei diese Gruppen durch ein bis fünf Halogenatome und/oder ein bis zwei 5-bis 6-gliedrige heterocyclische, aliphatische oder aromatische Reste, enthaltend ein bis drei Heteroatome, ausgewählt aus der Gruppe Sauerstoff, Schwefel oder Stickstoff, substituiert sind, die ein oder zwei der folgenden Substituenten tragen können: Halogen, C₁-C₃-Alkyl oder C₁-C₃-Alkoxy;
oder
R¹, R² gemeinsam eine Alkylenkette mit 4 bis 7 Gliedern, welche durch Sauerstoff, Schwefel oder N-Methyl unterbrochen sein kann;
R³-R⁶ Wasserstoff, C₁-C₄-Alkyl, C₁-C₄-Alkoxy, C₁-C₄-Halogenalkyl, C₁-C₄-Halogenalkoxy, C₁-C₄-Alkylthio, C₁-C₄-Halogenalkylthio, Halogen, Cyano oder Nitro;
X Sauerstoff oder Schwefel
oder dessen umweltverträglichen Salzes, behandelt.

## Claims

1. A quinoline-3-carboxamide of the general formula I 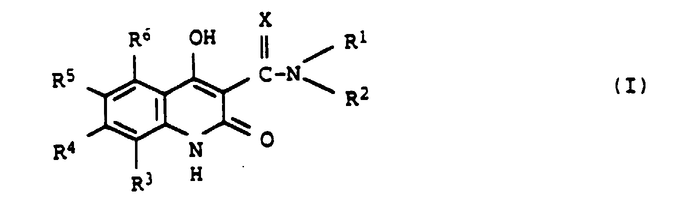 where the substituents have the following meanings:
R¹ is hydrogen, C₁-C₂₅-alkyl, C₃-C₂₅-alkenyl, C₃-C₂₅-alkynyl or C₃-C₈-cycloalkyl, where these groups can carry one to five halogen atoms and/or one to three of the following radicals: cyano, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl, phenylthio or phenoxy, where the phenyl radicals for their part can carry one to three of the following groups: C₁-C₄-alkyl, C₁-C₄-alkoxy, C₁-C₄-haloalkyl, C₁-C₄-haloalkoxy, halogen, cyano or nitro;
R² is hydrogen, hydroxyl, C₁-C₈-alkoxy, C₃-C₈-alkenyloxy, di-C₁-C₄-alkylamino, C₁-C₂₅-alkyl, C₃-C₂₅-alkenyl, C₃-C₂₅-alkynyl, or C₃-C₈-cycloalkyl, where said organic radicals can carry one to five halogen atoms and/or one to three of the following radicals: cyano, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl, phenylthio or phenoxy, where the phenyl radicals for their part can carry one to three of the following groups: C₁-C₄-alkyl, C₁-C₄-alkoxy, C₁-C₄-haloalkyl, C₁-C₄-haloalkoxy, halogen, cyano or nitro;
R² further is substituted C₁-C₈-alkoxy, C₃-C₈-alkenyloxy, di-C₁-C₄-alkylamino, C₁-C₂₅-alkyl, C₃-C₂₅-alkenyl, C₃-C₂₅-alkynyl or C₃-C₈-cycloalkyl, where these groups are substituted by one to five halogen atoms and/or one or two 5- to 6-membered heterocyclic, aliphatic or aromatic radicals, comprising one to three heteroatoms selected from the group consisting of oxygen, sulfur and nitrogen, which can carry one or two of the following substituents: halogen, C₁-C₃-alkyl or C₁-C₃-alkoxy;
or
R¹ and R² together are an alkylene chain having 4 to 7 members, which can be interrupted by oxygen, sulfur or N-methyl;
R³-R⁶ are hydrogen, C₁-C₄-alkyl, C₁-C₄-alkoxy, C₁-C₄-haloalkyl, C₁-C₄-haloalkoxy, C₁-C₄-alkylthio, C₁-C₄-haloalkylthio, halogen, cyano or nitro;
X is oxygen or sulfur;
and their environmentally tolerable salts,
with the proviso that R² is not hydrogen, C₁-C₃-alkyl, n-butyl, 3-methylbutyl, cyclohexyl, hexyl, heptyl, octyl, 2-chlorobenzyl, 3-dimethylaminopropyl, 3-diethylaminopropyl, 2-morpholinoethyl and 2-(3,4-dimethoxyphenyl)ethyl if R¹ and R³ to R⁶ are hydrogen and X is oxygen and R² is not benzyl if R¹ is methyl, R³ to R⁶ are hydrogen and X is oxygen, and also R¹ and R² together are not morpholino if R³ to R⁶ are hydrogen and X is oxygen; further excluding the compounds 7-chloro-2,4-dioxo-3-(1,2,3,4-tetrahydroquinoline)hydroxamic acid and 7-chloro-5-iodo-2,4-dioxo-3-(1,2,3,4-tetrahydroquinoline)hydroxamic acid.

2. A quinoline-3-carboxamide of the formula I as claimed in claim 1, where X is oxygen or sulfur, R¹ is hydrogen and
R² is branched or unbranched C₁-C₁₅-alkyl, C₃-C₁₅-alkenyl, C₃-C₁₅-alkynyl, C₁-C₅-alkoxy or C₃-C₆-cycloalkyl, where said radicals can be substituted by halogen, C₁-C₃-alkoxy, C₁-C₃-haloalkoxy or unsubstituted or substituted phenyl.

3. A process for preparing the quinoline-3-carboxamides I as claimed in claim 1, which comprises reacting a quinoline-3-carboxylic acid derivative of the formula II 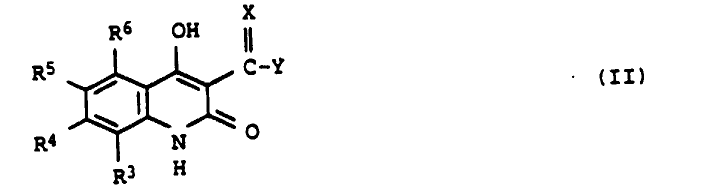 where X and R³ to R⁶ have the meanings indicated in claim 1 and Y is hydroxyl, halogen or an alkoxy group, in a manner known per se with a substituted amine of the formula III

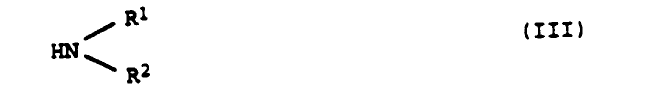

4. A process for preparing the quinoline-3-carboxamides of the formula I as claimed in claim 1, where X is oxygen and R¹ is hydrogen, which comprises reacting a 2,4-dihydroxyquinoline of the formula IV 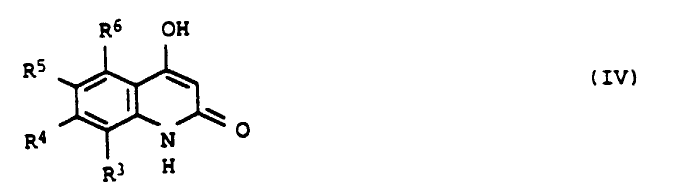 with an isocyanate of the formula V
O=C=N-R² (V).

5. A herbicidal composition, comprising a quinoline-3-carboxamide of the formula I as claimed in claim 1 and inert additives.

6. A method of controlling undesired vegetation, which comprises treating the undesired plants and/or their habitat with a herbicidally active amount of a quinoline-3-carboxamide of the formula I 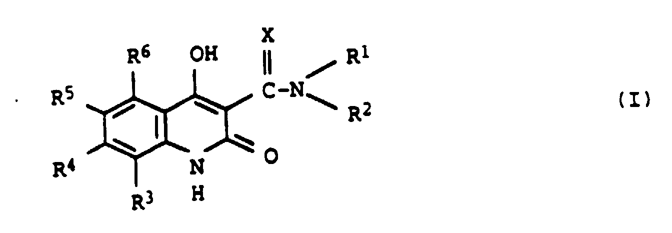 where the substituents have the following meanings:
R¹ is hydrogen, C₁-C₂₅-alkyl, C₃-C₂₅-alkenyl, C₃-C₂₅-alkynyl or C₃-C₈-cycloalkyl, where these groups can carry one to five halogen atoms and/or one to three of the following radicals: cyano, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl, phenylthio or phenoxy, where the phenyl radicals for their part can carry one to three of the following groups: C₁-C₄-alkyl, C₁-C₄-alkoxy, C₁-C₄-haloalkyl, C₁-C₄-haloalkoxy, halogen, cyano or nitro;
R² is hydrogen, hydroxyl, C₁-C₈-alkoxy, C₃-C₈-alkenyloxy, di-C₁-C₄-alkylamino, C₁-C₂₅-alkyl, C₃-C₂₅-alkenyl, C₃-C₂₅-alkynyl, or C₃-C₈-cycloalkyl, where said organic radicals can carry one to five halogen atoms and/or one to three of the following radicals: cyano, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl, phenylthio or phenoxy, where the phenyl radicals for their part can carry one to three of the following groups: C₁-C₄-alkyl, C₁-C₄-alkoxy, C₁-C₄-haloalkyl, C₁-C₄-haloalkoxy, halogen, cyano or nitro;
R² further is substituted C₁-C₈-alkoxy, C₃-C₈-alkenyloxy, di-C₁-C₄-alkylamino, C₁-C₂₅-alkyl, C₃-C₂₅-alkenyl, C₃-C₂₅-alkynyl or C₃-C₈-cycloalkyl, where these groups are substituted by one to five halogen atoms and/or one or two 5- to 6-membered heterocyclic, aliphatic or aromatic radicals, comprising one to three heteroatoms selected from the group consisting of oxygen, sulfur and nitrogen, which can carry one or two of the following substituents: halogen, C₁-C₃-alkyl or C₁-C₃-alkoxy;
or
R¹ and R² together are an alkylene chain having 4 to 7 members, which can be interrupted by oxygen, sulfur or N-methyl;
R³-R⁶ are hydrogen, C₁-C₄-alkyl, C₁-C₄-alkoxy, C₁-C₄-haloalkyl, C₁-C₄-haloalkoxy, C₁-C₄-alkylthio, C₁-C₄-haloalkylthio, halogen, cyano or nitro;
X is oxygen or sulfur
or its environmentally tolerable salt.

## Revendications

1. Quinoléine-3-carboxamides de formule générale I 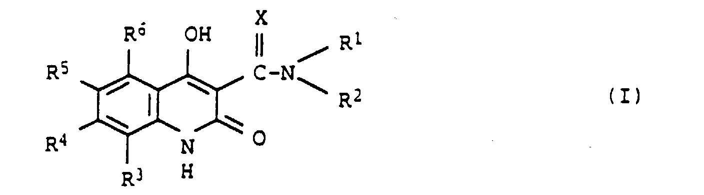 dans laquelle les symboles ont les significations suivantes :
R¹ représente l'hydrogène, un groupe alkyle en C1-C25, alcényle en C3-C25, alcynyle en C3-C25, cycloalkyle en C3-C8, ces groupes pouvant porter un à cinq atomes d'halogènes et/ou un à trois des groupes suivants : cyano, cycloalkyle en C3-C8, alcoxy en C1-C4, halogénoalcoxy en C1-C4, alkylthio en C1-C4, halogénoalkylthio en C1-C4, di-(alkyle en Cl-C4)amino, phényle, phénylthio, phénoxy, les radicaux phényle pouvant eux-mêmes porter un à trois des groupes suivants : alkyle en C1-C4, alcoxy en C1-C4, halogénoalkyle en C1-C4, halogénoalcoxy en C1-C4, halogéno, cyano ou nitro ;
R² représente l'hydrogène, un groupe hydroxy, alcoxy en C1-C8, alcényloxy en C3-C8, di-(alkyle en C1-C4)amine, alkyle en C1-C25, alcényle en C3-C25, alcynyle en C3-C25, cycloalkyle en C3-C8, les radicaux organiques mentionnés pouvant porter un à cinq atomes d'halogènes et/ou un à trois des radicaux suivants : cyano, cycloalkyle en C3-C8, alcoxy en C1-C4, halogénoalcoxy en C1-C4, alkylthio en C1-C4, halogénoalkylthio en C1-C4, di-(alkyle en C1-C4)amine, phényle, phénylthio, phénoxy, les groupes phényle pouvant eux-mêmes porter un à trois des substituants suivants : alkyle en C1-C4, alcoxy en C1-C4, halogénoalkyle en C1-C4, halogénoalcoxy en C1-C4, halogéno, cyano ou nitro ;
R² peut en outre représenter un groupe alcoxy en C1-C8, alcényloxy en C3-C8, di-(alkyle en C1-C4)amine, alkyle en C1-C25, alcényle en C3-C25, alcynyle en C3-C25 ou cycloalkyle en C3-C8 substitué, ces groupes étant substitués par un à cinq atomes d'halogènes et/ou un à deux radicaux hétérocycliques, aliphatiques ou aromatiques, à cinq ou six chaînons, contenant un à trois hétéroatomes choisis parmi l'oxygène, le soufre et l'azote, et qui peuvent porter un ou deux des substituants suivants : halogéno, alkyle en C1-C3 ou alcoxy en C1-C3 ;
ou bien
R¹, R² forment ensemble une chaîne alkylène de 4 à 7 chaînons qui peut être interrompue par l'oxygène, le soufre ou un groupe N-méthyle ;
R³ à R⁶ représentent l'hydrogène, des groupes alkyle en C1-C4, alcoxy en C1-C4, halogénoalkyle en C1-C4, halogénoalcoxy en C1-C4, alkylthio en C1-C4, halogénoalkylthio en C1-C4, des halogènes, des groupes cyano ou nitro ;
X représente l'oxygène ou le soufre ;
et leurs sels non polluants,
sous réserve que R² ne peut représenter l'hydrogène, un groupe alkyle en C1-C3, n-butyle, 3-méthylbutyle, cyclohexyle, hexyle, heptyle, octyle, 2-chlorobenzyle, 3-(diméthylamino)propyle, 3-(diéthylamino)propyle, 2-morpholinoéthyle ou 2-(3,4-diméthoxyphényl)éthyle lorsque R¹ et R³ à R⁶ représentent l'hydrogène et X l'oxygène, et que R² ne peut représenter un groupe benzyle lorsque R¹ représente un groupe méthyle, R³ à R⁶ l'hydrogène et X l'oxygène, et R¹ et R² ne peuvent représenter ensemble un groupe morpholino lorsque R³ à R⁶ représentent l'hydrogène et X l'oxygène ; à l'exception en outre des composés acide 7-chloro-2,4-dioxo-3-(1,2,3,4-tétrahydroquinoléine)hydroxamique et acide 7-chloro-5-iodo-2,4-dioxo-3-(1,2,3,4-tétrahydroquinoléine)hydroxamique.

2. Quinoléine-3-carboxamides de formule I de la revendication 1 dans laquelle X représente l'oxygène ou le soufre, R¹ l'hydrogène et
R² un groupe alkyle en C1-C15 à chaîne droite ou ramifié, alcényle en C3-C15, alcynyle en C3-C15, alcoxy en C1-C5, cycloalkyle en C3-C6, ces groupes pouvant éventuellement être substitués par des halogènes, des groupes alcoxy en C1-C3, halogénoalcoxy en C1-C3 ou phényle lui-même éventuellement substitué.

3. Procédé de préparation des quinoléine-3-carboxamides I de la revendication 1, caractérisé par le fait que l'on fait réagir de manière connue en soi un dérivé d'acide quinoléine-3-carboxylique de formule II 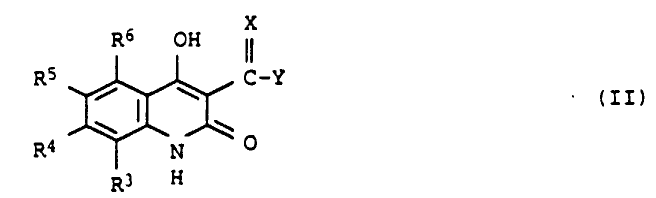 dans laquelle X et R³ à R⁶ ont les significations indiquées dans la revendication 1 et Y représente un groupe hydroxy, un halogène ou un groupe alcoxy, avec une amine substituée de formule III

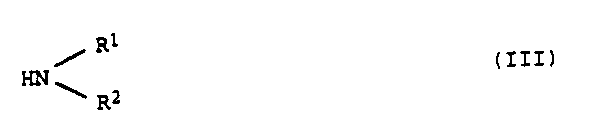

4. Procédé de préparation des quinoléine-3-carboxamides de formule I de la revendication 1 dans laquelle X représent l'oxygène et R¹ l'hydrogène, caractérisé par le fait que l'on fait réagir une 2,4-dihydroxyaquinoléine de formule IV 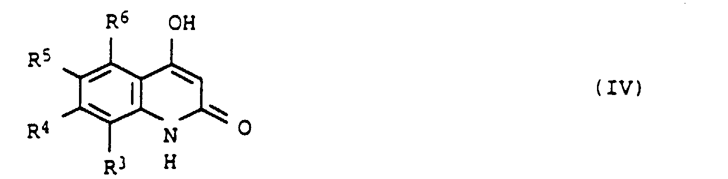 avec un isocyanate de formule V
O=C=N-R² (V)

5. Produit herbicide contenant un quinoléine-3-carboxamide de formule I de la revendication 1 et des additifs inertes.

6. Procédé pour combattre les croissances de végétaux indésirables, caractérisé par le fait que l'on traite les végétaux indésirables et/ou leur habitat par une quantité herbicide efficace d'un quinoléine-3-carboxamide de formule I 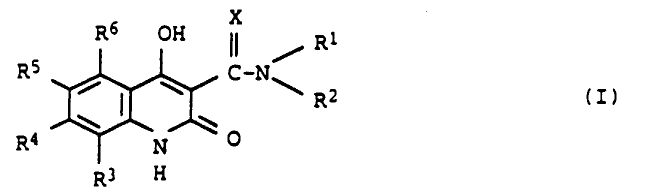 dans laquelle les symboles ont les significations suivantes :
R¹ représente l'hydrogène, un groupe alkyle en C1-C25, alcényle en C3-C25, alcynyle en C3-C25, cycloalkyle en C3-C8, ces groupes pouvant porter un à cinq atomes d'halogènes et/ou un à trois des groupes suivants : cyano, cycloalkyle en C3-C8, alcoxy en C1-C4, halogénoalcoxy en C1-C4, alkylthio en C1-C4, halogénoalkylthio en C1-C4, di-(alkyle en Cl-C4)amino, phényle, phénylthio, phénoxy, les radicaux phényle pouvant eux-mêmes porter un à trois des groupes suivants : alkyle en C1-C4, alcoxy en C1-C4, halogénoalkyle en C1-C4, halogénoalcoxy en C1-C4, halogéno, cyano ou nitro ;
R² représente l'hydrogène, un groupe hydroxy, alcoxy en C1-C8, alcényloxy en C3-C8, di-(alkyle en C1-C4)amino, alkyle en C1-C25, alcényle en C3-C25, alcynyle en C3-C25, cycloalkyle en C3-C8, les radicaux organiques mentionnés pouvant porter un à cinq atomes d'halogènes et/ou un à trois des radicaux suivants : cyano, cycloalkyle en C3-C8, alcoxy en C1-C4, halogénoalcoxy en C1-C4, alkylthio en C1-C4, halogénoalkylthio en C1-C4, di-(alkyle en C1-C4)amino, phényle, phénylthio, phénoxy, les groupes phényle pouvant eux-mêmes porter un à trois des substituants suivants : alkyle en C1-C4, alcoxy en C1-C4, halogénoalkyle en C1-C4, halogénoalcoxy en C1-C4, halogéno, cyano ou nitro ;
R² peut en outre représenter un groupe alcoxy en C1-C8, alcényloxy en C3-C8, di-(alkyle en C1-C4)amino, alkyle en C1-C25, alcényle en C3-C25, alcynyle en C3-C25 ou cycloalkyle en C3-C8 substitué, ces groupes étant substitués par un à cinq atomes d'halogènes et/ou un à deux radicaux hétérocycliques, aliphatiques ou aromatiques, à cinq ou six chaînons, contenant un à trois hétéroatomes choisis parmi l'oxygène, le soufre et l'azote, et qui peuvent porter un ou deux des substituants suivants : halogéno, alkyle en C1-C3 ou alcoxy en C1-C3 ;
ou bien
R¹, R² forment ensemble une chaîne alkylène de 4 à 7 chaînons qui peut être interrompue par l'oxygène, le soufre ou un groupe N-méthyle ;
R³ à R⁶ représentent l'hydrogène, des groupes alkyle en C1-C4, alcoxy en C1-C4, halogénoalkyle en C1-C4, halogénoalcoxy en C1-C4, alkylthio en C1-C4, halogénoalkylthio en C1-C4, des halogènes, des groupes cyano ou nitro ;
X représente l'oxygène ou le soufre ;
et leurs sels non polluants.
